(19) Europäisches Patentamt
European Patent Office
Office européen des brevets

(11) EP 1 146 041 B1

(12) # FASCICULE DE BREVET EUROPEEN

(45) Date de publication et mention de la délivrance du brevet:
**12.11.2003 Bulletin 2003/46**

(21) Numéro de dépôt: **01400940.1**

(22) Date de dépôt: **12.04.2001**

(51) Int Cl.[7]: **C07D 209/70**, A61K 31/403, A61P 43/00, C07D 307/93, C07D 333/78, C07D 317/70, C07C 215/42, C07D 319/14, C07D 209/16, C07D 407/12, C07D 209/40

(54) **Dérivés d'hétérocycloalkylbenzocyclobutane et d'hétéroarylbenzocyclobutane et leur utilisation en tant qu'inhibiteurs de recapture de la sérotonine et de la noradrénaline**

Derivate von Heterocycloalkylbenzocyclobutan und Heteroarylbenzocyclobutan und deren Verwendung als Inhibitoren der Wiederaufnahme von Serotonin und Noradrenalin

Derivatives of heterocycloalkylbenzocyclobutane and heteroarylbenzocyclobutane and their use as inhibitors of the recapture of serotonine and of noradrenaline

(84) Etats contractants désignés:
**AT BE CH CY DE DK ES FI FR GB GR IE IT LI LU MC NL PT SE TR**
Etats d'extension désignés:
**AL LT LV MK RO SI**

(30) Priorité: **13.04.2000 FR 0004742**

(43) Date de publication de la demande:
**17.10.2001 Bulletin 2001/42**

(73) Titulaire: **LES LABORATOIRES SERVIER**
**92200 Neuilly sur Seine (FR)**

(72) Inventeurs:
- **Peglion, Jean-Louis**
  **78110 Le Vesinet (FR)**
- **Dessinges, Aimée**
  **92500 Rueil Malmaison (FR)**
- **Goument, Bertrand**
  **78220 Viroflay (FR)**
- **Millan, Mark**
  **78230 Le Pecq (FR)**
- **Lejeune, Francoise**
  **92210 Saint Cloud (FR)**
- **Brocco, Mauricette**
  **75003 Paris (FR)**

(56) Documents cités:
**EP-A- 0 940 386** **US-A- 5 530 013**

Remarques:
Le dossier contient des informations techniques présentées postérieurement au dépôt de la demande et ne figurant pas dans le présent fascicule.

EP 1 146 041 B1

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Printed by Jouve, 75001 PARIS (FR)

## Description

La présente invention concerne de nouveaux dérivés d'hétérocycloalkylbenzocyclobutane et d'hétéroarylbenzocyclobutane, leur procédé de préparation et les compositions pharmaceutiques qui les contiennent.

Des dérivés bicycliques de benzocyclobutane ont été décrits dans la demande EP 0 940 386 en tant qu'inhibiteurs de la recapture de sérotonine et de noradrénaline.

Les composés de la présente invention agissent comme de puissants inhibiteurs de recapture de la sérotonine et de la noradrénaline. A ce titre, ils sont utiles, en tant que médicament, pour le traitement de la dépression, des attaques de panique, des troubles obsessionnels compulsifs, des phobies, des troubles impulsifs, de l'abus de drogue, de l'anxiété, de l'obésité et de la boulimie.
En effet, les produits de la présente invention se sont montrés actifs d'une part, in vitro, sur le test de caractérisation de l'inhibition de la recapture de sérotonine et de noradrénaline, et d'autre part, in vivo. Ainsi, dans les expériences de microdialyse, réalisées dans le cortex frontal chez le rat, les composés de l'invention entraînent dans ce territoire une augmentation de la libération de sérotonine, de noradrénaline et de dopamine. Les composés revendiqués par la Demanderesse sont donc tout à fait aptes à être utilisés dans des pathologies qui sont liés à un défaut de transmission de ces deux neuromédiateurs. Cet effet particulièrement intéressant des composés de l'invention est aussi démontré lors du test d'enfouissement des billes chez la souris.

Plus particulièrement, la présente invention concerne les composés de formule **(I)** :

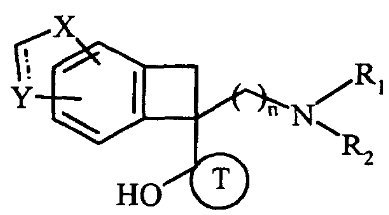

**(I)**

dans laquelle :

- ----- représente une liaison simple ou une liaison double,
- n représente 1,
- $R_1$, $R_2$, identiques ou différents, indépendamment l'un de l'autre représentent un groupement choisi parmi atome d'hydrogène, groupement alkyle ($C_1$-$C_6$) linéaire ou ramifié, 2,3-dihydro-1,4-benzodioxin-2-ylméthyle,
- X représente un groupement choisi parmi -CH=CH-, atome d'oxygène, groupement $S(O)_m$ dans lequel m est un entier compris entre 0 et 2 inclus, et $NR_3$ dans lequel $R_3$ représente un groupement choisi parmi atome d'hydrogène, groupement alkyle ($C_1$-$C_6$) linéaire ou ramifié, aryle, arylalkyle ($C_1$-$C_6$) linéaire ou ramifié, cycloalkyle, cycloalkylalkyle ($C_1$-$C_6$) linéaire ou ramifié, alkényle ($C_2$-$C_6$) linéaire ou ramifié, et alkynyle ($C_2$-$C_6$) linéaire ou ramifié,
- Y représente un groupement CH ou $CH_2$ selon que ------- représente une liaison simple ou une liaison double, ou peut prendre la définition supplémentaire atome d'oxygène quand X représente un atome d'oxygène,
- T représente un groupement cyclopentyle ou cyclohexyle,
- leurs isomères optiques ainsi que leurs sels d'addition à un acide ou à une base pharmaceutiquement acceptable.

Par groupement aryle, on comprend un groupement choisi parmi phényle, biphényle, naphtyle, dihydronaphtyle, tétrahydronaphtyle, indanyle, indényle, et benzocyclobutyle, chacun de ces groupements pouvant être éventuellement substitués par un ou plusieurs groupements, identiques ou différents, choisis parmi atomes d'halogène, groupements alkyle ($C_1$-$C_6$) linéaire ou ramifié, hydroxy, alkoxy ($C_1$-$C_6$) linéaire ou ramifié, nitro, cyano, trihalogénoalkyle ($C_1$-$C_6$) linéaire ou ramifié, amino, monoalkylamino et dialkylamino ($C_1$-$C_6$) linéaire ou ramifié.

Par groupement cycloalkyle, on comprend un système mono ou polycyclique, de 3 à 12 chaînons, contenant éventuellement une ou plusieurs insaturations, celles-ci ne conférant pas de caractère aromatique audit système cyclique.

Selon une variante avantageuse de l'invention, les composés préférés de l'invention sont les composés de formule **(I/A)** :

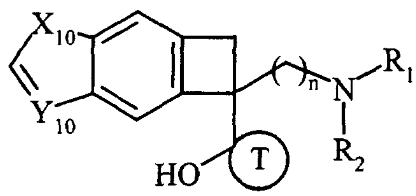

**(I/A)**

dans laquelle n, $R_1$, $R_2$ et T sont tels que définis dans la formule (I), $X_{10}$ représente un atome d'oxygène ou un atome de soufre, et $Y_{10}$ représente un groupement CH.

Selon une autre variante avantageuse de l'invention, les composés préférés de l'invention sont les composés de formule **(I/B):**

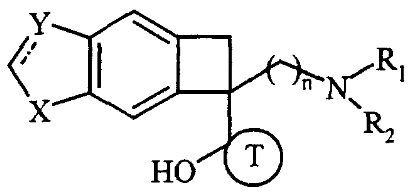

**(I/B)**

dans laquelle X, Y, n, $R_1$, $R_2$, $R_3$ et T sont tels que définis dans la formule (I).

D'une façon intéressante, les composés préférés de l'invention sont les composés de formule (I/B) dans laquelle n est égal à 1, X représente un groupement $NR_3$ dans lequel $R_3$ représente un atome d'hydrogène ou un groupement alkyle ($C_1$-$C_6$) linéaire ou ramifié.

Selon une troisième variante avantageuse de l'invention, les composés préférés de l'invention sont les composés de formule **(I/C) :**

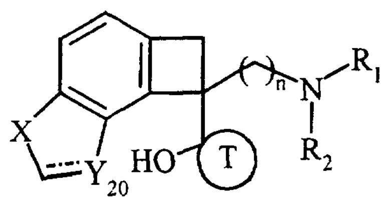

**(I/C)**

dans laquelle n, $R_1$, $R_2$, X et T sont tels que définis dans la formule (I), et $Y_{20}$ représente un groupement CH ou $CH_2$.

Selon une quatrième variante avantageuse de l'invention, les composés préférés de l'invention sont les composés de formule **(I/D) :**

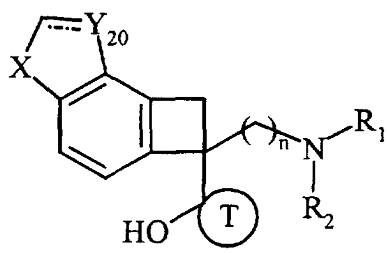

**(I/D)**

dans laquelle n, $R_1$, $R_2$, X et T sont tels que définis dans la formule (I), et $Y_{20}$ représente un groupement CH ou $CH_2$.

Selon une autre variante avantageuse de l'invention, les composés préférés de l'invention sont les composés de formule **(I/E) :**

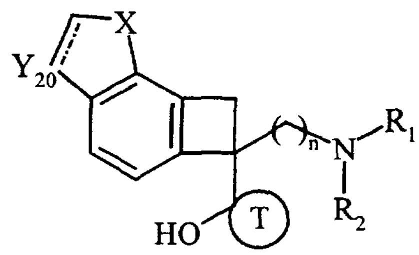

**(I/E)**

dans laquelle n, $R_1$, $R_2$, X et T sont tels que définis dans la formule (I), et $Y_{20}$ représente un groupement CH ou $CH_2$.

Enfin, selon une dernière variante intéressante de l'invention, les composés préférés de l'invention sont les composés de formule **(I/F) :**

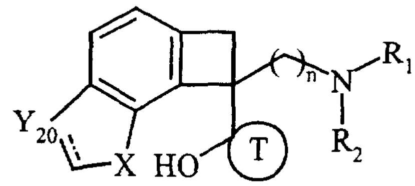

**(I/F)**

dans laquelle n, $R_1$, $R_2$, X et T sont tels que définis dans la formule (I), et $Y_{20}$ représente un groupement CH ou $CH_2$.

Les composés préférés de l'invention sont le :

- 1-{6-[(diméthylamino)méthyl]-1-méthyl-2,3,5,6-tétrahydro-1*H*-cyclobuta[*f*]indol-6-yl} cyclohexanol,
- 1-{6-[(diméthylamino)méthyl]-2,3,5,6-tétrahydro-1*H*-cyclobuta[*f*]indol-6-yl} cyclohexanol,
- 1-{5-[(diméthylamino)méthyl]-5,6-dihydrocyclobuta[*f*][1]benzofuran-5-yl} cyclohexanol,
- 1-{5-[(diméthylamino)méthyl]-5,6-dihydrocyclobuta[*f*][*l*]benzothien-5-yl} cyclohexanol,
- 1-{6-[(diméthylamino)méthyl]-1-méthyl-5,6-dihydro-1*H*-cyclobuta[*f*]indol-6-yl} cyclohexanol,
- 1-{7-[(diméthylamino)méthyl]-2,3,6,7-tétrahydro-1*H*-cyclobuta[*e*]indol-7-yl} cyclohexanol,
- 1-{5-[(méthylamino)méthyl]-5,6-dihydrocyclobuta[*f*][1]benzothièn-5-yl} cyclopentanol,
- 1-{5-[(diméthylamino)méthyl]-5,6-dihydrocyclobuta[*f*][1]benzothièn-5-yl} cyclopentanol,
- (+) 1-{5-[(diméthylamino)méthyl]-5,6-dihydrocyclobuta[*f*][1]benzothièn-5-yl} cyclopentanol,
- (-) 1-{5-[(diméthylamino)méthyl]-5,6-dihydrocyclobuta[*f*][1]benzothièn-5-yl} cyclopentanol,
- 1-{6-[(diméthylamino)méthyl]-5,6-dihydrocyclobuta[*f*][1]benzothièn-6-yl} cyclopentanol,
- 1-{5-[(diméthylamino)méthyl]-1-méthyl-5,6-dihydra-1*H*-cyclobuta[*f*]indol-5-yl} cyclopentanol,
- 1-{7-[(diméthylamino}méthyl]-6,7-dihydrocyclobuta[*g*][1]benzofuran-7-yl} cyclopentanol,
- 1-{1-[(diméthylamino)méthyl]-1,2-dihydrocyclobuta[*b*]naphthalèn-1-yl}cyclopentanol,
- 1-{7-[(diméthylamino)méthyl]-6,7-dihydro-3*H*-cyclobuta[*e*]indol-7-yl}cyclopentanol,
- 1-{1-[(dimethylamino)methyl]-1,2-dihydrocyclobuta[*a*]naphthalèn-1-yl}cyclopentanol.

Les isomères ainsi que les sels d'addition à un acide ou à une base pharmaceutiquement acceptable des composés préférés font partie intégrante de l'invention.

Par isomères, on comprend les isomères optiques tels que les diastéréoisomères et les énantiomères.

Parmi les acides pharmaceutiquement acceptables, on peut citer à titre non limitatif les acides chlorhydrique, bromhydrique, sulfurique, phosphonique, acétique, trifluoroacétique, lactique, pyruvique, malonique, succinique, glutarique, fumarique, tartrique, maléïque, citrique, ascorbique, oxalique, méthane sulfonique, camphorique, etc...

Parmi les bases pharmaceutiquement acceptables, on peut citer à titre non limitatif l'hydroxyde de sodium, l'hydroxyde de potassium, la triéthylamine, la tertbutylamine, etc...

La présente invention concerne aussi le procédé de préparation des composés de formule (I), caractérisé en ce qu'on utilise comme produit de départ :

***a) soit*** un composé de formule **(II) :**

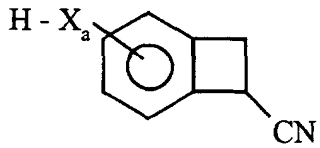

**(II)**

dans laquelle $X_a$ représente un atome de soufre, d'oxygène, ou un groupement NH, composé de formule (II) que l'on fait réagir avec un composé de formule **(III) :**

$$Z - CH(OA)_2 \qquad \textbf{(III)}$$

dans laquelle A représente un groupement alkyle ($C_1$-$C_4$) linéaire ou ramifié et Z représente un groupement formyle (dans le cas où $X_a$ représente un groupement NH), ou un groupement $-CH_2Hal$ dans lequel Hal représente un atome de chlore, de brome ou d'iode,
pour conduire aux composés de formule **(IV) :**

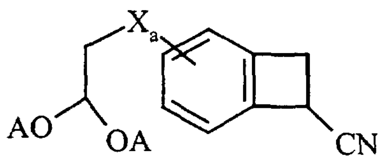

**(IV)**

dans laquelle $X_a$ et A sont tels que définis précédemment, composés de formule (IV) qui sont soumis :

- soit dans le cas où $X_a$ prend la définition $X_1$ représentant un atome d'oxygène ou un atome de soufre, à des conditions de cyclisation par action d'un acide tel que l'acide

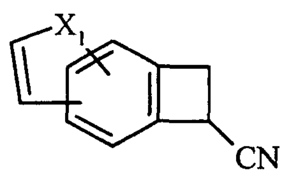

**(V/a)**

dans laquelle $X_1$ représente un atome d'oxygène ou de soufre,
composés de formule (V/a) qui sont traités :
en présence d'une base forte, par une cétone cyclique de formule **(VI) :**

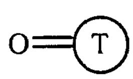

**(VI)**

dans laquelle T a les mêmes significations que dans la formule (I),
pour conduire aux composés de formule **(VII/a),**

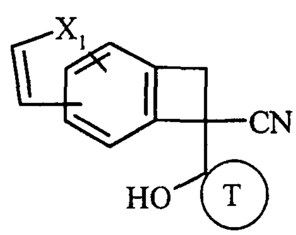

**(VII/a)**

dans laquelle $X_1$ et T sont tels que définis précédemment,
composés de formule (VII/a) qui sont soumis à l'action d'un agent réducteur selon des conditions classiques de la synthèse organique, pour conduire aux composés de formule **(I/a),** cas particulier des composés de formule (I)

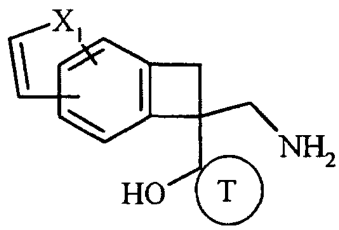

**(I/a)**

dans laquelle $X_1$ et T sont tels que définis précédemment,
composés de formule (I/a) pour lesquels :

⇨ ***soit*** on substitue la fonction amine primaire selon des méthodes classiques de la synthèse organique, telle que l'amination réductrice ou la substitution nucléophile de dérivé de formule **(VIIIa) :**

$$R'_1 - Z_1 \qquad \textbf{(VIIIa)}$$

dans laquelle $R'_1$ a les mêmes définitions que $R_1$ dans la formule (I) exceptées la valeur dans laquelle $R'_1$ a les mêmes définitions que $R_1$ dans la formule (I) exceptées la valeur atome d'hydrogène, et $Z_1$ représente un groupe partant usuel de la chimie organique tel que atome d'halogène, groupement mésylate ou tosylate,
pour conduire aux composés de formule **(I/b$_1$),** cas particulier des composés de formule (I) :

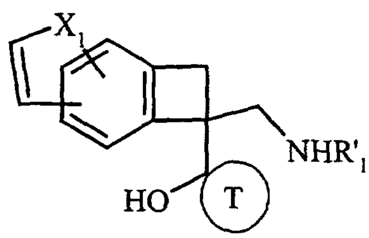

**(I/b$_1$)**

dans laquelle $X_1$, T et $R'_1$ sont tels que définis précédemment,

⇨ ***soit*** selon une variante avantageuse du procédé, on traite par un dérivé de formule **(VIIIb) :**

$$R''_1 - COZ_2 \qquad \textbf{(VIIIb)}$$

dans laquelle $R''_1$ représente un groupement hétérocycloalkylalkyle ($C_1$-$C_5$) linéaire ou ramifié, et $Z_2$ représente un atome de chlore ou un groupement imidazolyle, pour conduire aux composés de formule **(I/b$_2$) :**

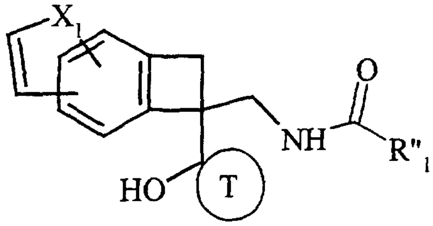

**(I/b$_2$)**

dans laquelle $X_1$, T et $R''_1$ sont tels que définis précédemment,
composés de formule ($I/b_2$) qui sont réduits par un agent réducteur classiquement utilisé en chimie organique, pour conduire aux composés de formule **($I/b_3$) :**

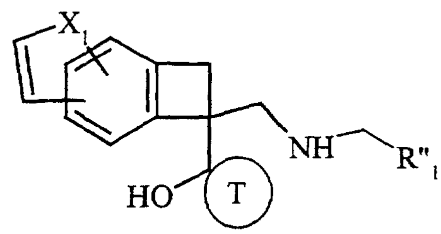

**($I/b_3$)**

dans laquelle $X_1$, $R''_1$ et T sont tels que définis précédemment,
l'ensemble des composés de formule ($I/b_1$) et ($I/b_3$) forment les composés de formule (I/b), composés de formule (I/b) qui sont traités selon les mêmes conditions que décrites précédemment par un composé de formule **(VIIIc) :**

$$R'_2 - Z_1 \qquad \textbf{(VIIIc)}$$

dans laquelle $Z_1$ est tel que défini précédemment et $R'_2$ prend les mêmes définitions que $R_2$ dans la formule (I) à l'exception de la valeur atome d'hydrogène,
pour conduire aux composés de formule **(I/c),** cas particulier des composés de formule (I) :

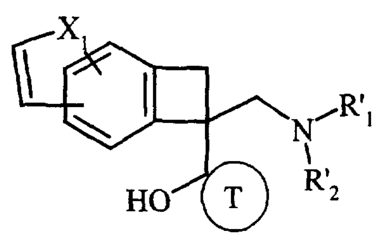

**(I/c)**

dans laquelle $X_1$, T, $R'_1$ et $R'_2$ sont tels que définis précédemment,
l'ensemble des composés de formule (I/a), (I/b), et (I/c), dans le cas particulier où $X_1$ représente un atome de soufre, forment les composés de formule **(I/e) :**

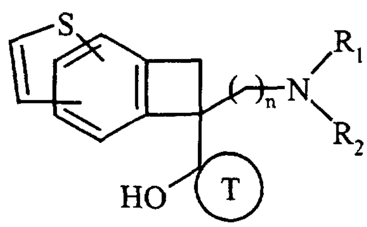

**(I/e)**

dans laquelle n, $R_1$, $R_2$ et T sont tels que définis dans la formule (I),
composés de formule (I/e) qui sont soumis à l'action d'un agent d'oxydation selon des conditions classiques de la synthèse organique, pour conduire aux composés de formule **(I/f),** cas particulier des composés de formule (I) :

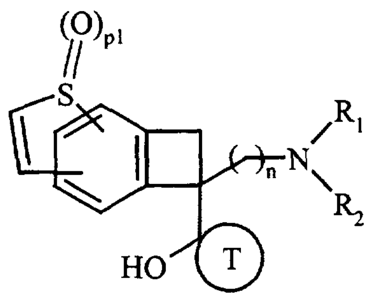

**(I/f)**

dans laquelle $p_1$ est un entier choisi parmi 1 et 2, et n, $R_1$, $R_2$ et T sont tels que définis précédemment, l'ensemble des composés de formule (I/a), (I/b), (I/c), (dans lesquels $X_1$ représente un atome d'oxygène ou de soufre) et (I/f) forment les composés de formule **(I/g) :**

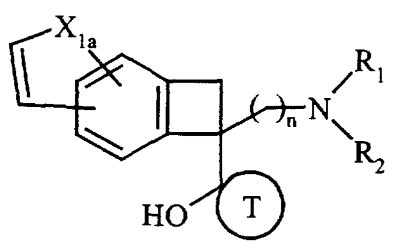

**(I/g)**

dans laquelle $X_{1a}$ représente un atome d'oxygène ou un groupement de formule $S(O)_p$ avec p tel que défini dans la formule (I), et n, $R_1$, $R_2$ et T sont tels que définis dans la formule (I),
composés de formule (I/g) qui sont soumis à l'action d'un agent réducteur selon des conditions classiques de la synthèse organique, pour conduire aux composés de formule **(I/h),** cas particulier des composés de formule (I) :

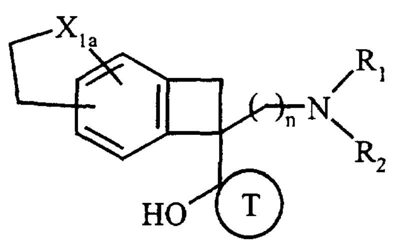

**(I/h)**

dans laquelle $X_{1a}$, n, $R_1$, $R_2$ et T sont tels que définis précédemment,

- soit, dans le cas où $X_a$ prend la définition $X'_2$ représentant un groupement NH, à l'action d'un chlorure d'acide sulfonique de formule **(XVI) :**

$$E - SO_2Cl \qquad \textbf{(XVI)}$$

dans laquelle E représente un groupement alkyle ($C_1$-$C_4$) linéaire ou ramifié, phényle ou p-toluyle, pour conduire aux composés de formule **(XVII) :**

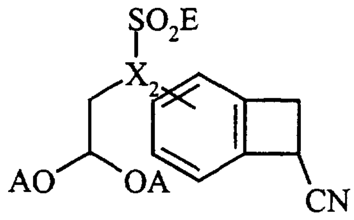

**(XVII)**

dans laquelle $X_2$ représente un atome d'azote, E et A sont tels que définis précédemment, composés de formule (XVII) qui sont cyclisés par action d'un acide pour conduire aux composés de formule **(V/b) :**

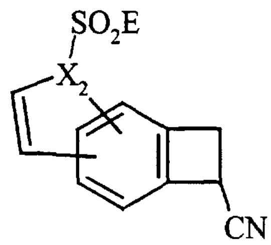

**(V/b)**

dans laquelle $X_2$ et E sont tels que définis précédemment,
composés de formule (V/b) dont on déprotège l'amine cyclique par action d'un agent basique, puis que l'on soumet à l'action d'un agent réducteur selon des conditions classiques de la synthèse organique, pour conduire aux composés de formule **(V/c) :**

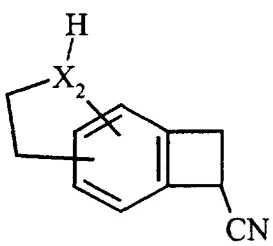

**(V/c)**

dans laquelle $X_2$ est tel que défini précédemment,
composés de formule (V/c) qui sont soumis à l'action d'un composé de formule (VI) tel que décrit précédemment pour conduire aux composés de formule **(VII/b) :**

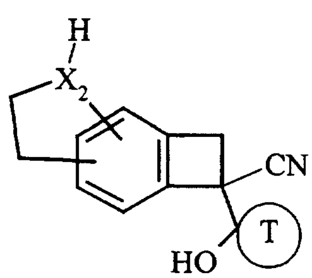

**(VII/b)**

dans laquelle $X_2$ est tel que défini précédemment et T est tel que défini dans la formule (I), composés de formule (VII/b) qui sont :

- ♦ *soit* traités selon les mêmes conditions que celles décrites pour les composés de formule (VII/a), pour conduire aux composés de formule **(I/i),** cas particulier des composés de formule (I) :

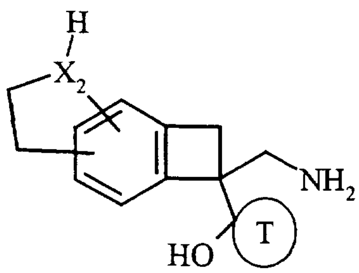

(I/i)

dans laquelle $X_2$ et T sont tels que définis précédemment, composés de formule (I/i) qui peuvent être traités successivement par un composé de formule (VIIIa) ou (VIIIb), puis (VIIIc), tels que définis précédemment, pour conduire respectivement aux composés de formule **(I/j)** et **(I/k),** cas particuliers des composés de formule (I):

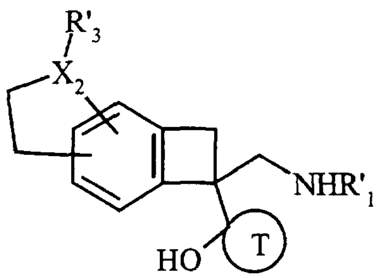

,

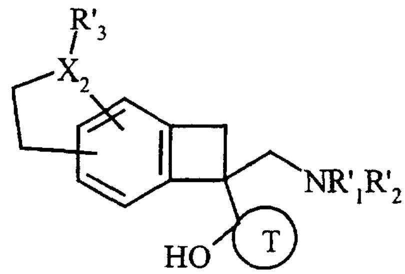

(I/j) (I/k)

dans lesquelles $X_2$, T, $R'_1$ et $R'_2$ sont tels que définis précédemment, et $R'_3$ a les mêmes définitions et valeurs que $R'_1$,
l'ensemble des composés de formules (I/i), (I/j), (I/k) forment les composés de formule **(I/l) :**

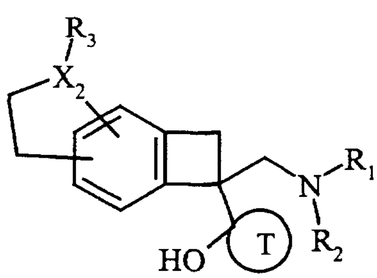

(I/l)

dans laquelle $X_2$, $R_1$, $R_2$, $R_3$ et T sont tels que définis dans la formule (I),
composés de formule (I/l) qui sont soumis à l'action d'un agent oxydant tel que le dioxyde de manganèse, pour conduire aux composés de formule **(I/m),** cas particulier des composés de formule (I) :

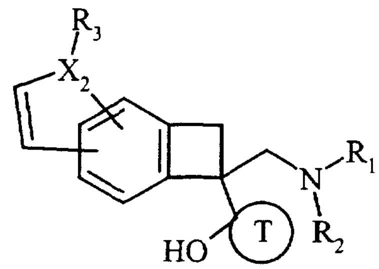

**(I/m)**

dans laquelle $X_2$, $R_1$, $R_2$, $R_3$ et T sont tels que définis précédemment,

- ***soit*** traités par un composé de formule (XVI) telle que définie précédemment, pour conduire aux composés de formule **(XVIII) :**

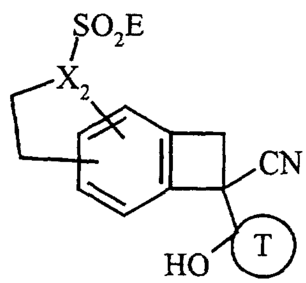

**(XVIII)**

dans laquelle T, $X_2$ et E sont tels que définis précédemment,
composés de formule (XVIII) qui sont réduits selon des conditions classiques de la synthèse organique, pour conduire aux composés de formule **(XIX) :**

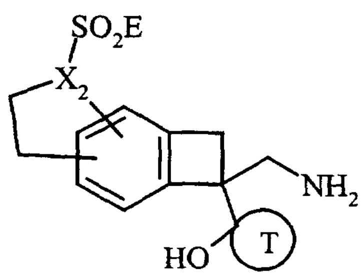

**(XIX)**

dans laquelle T, $X_2$ et E sont tels que définis précédemment,
composés de formule (XIX) dont la fonction amine primaire peut être substituée par action d'un composé de formule ($VIII_a$) tel que défini précédemment, pour conduire aux composés de formule **(XX) :**

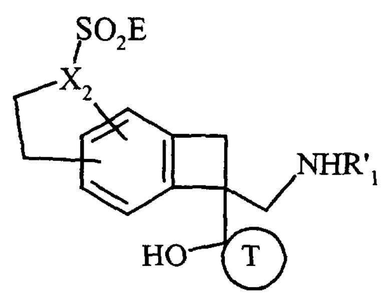

**(XX)**

dans laquelle T, $X_2$, E et $R'_1$ ont les mêmes significations que celles décrites précédemment,
composés de formule (XX) qui peuvent être transformés en amine tertiaire par action d'un composé de

formule ($VIII_b$) telle que définie précédemment, pour conduire aux composés de formule **(XXI) :**

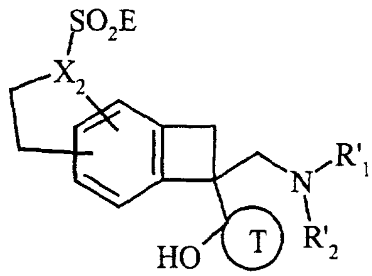

**(XXI)**

dans laquelle T, $X_2$, E, $R'_1$ et $R'_2$ sont tels que définis précédemment,
composés de formule (XX) et (XXI) qui sont ensuite déprotégés par traitement avec du sodium dans l'ammoniaque liquide, pour conduire respectivement aux composés de formules **(I/n)** et **(I/o),** cas particuliers des composés de formule (I) :

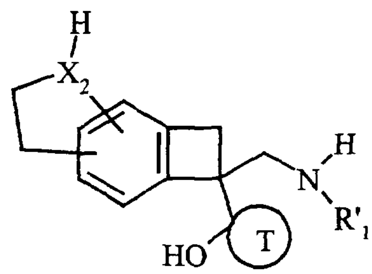

,

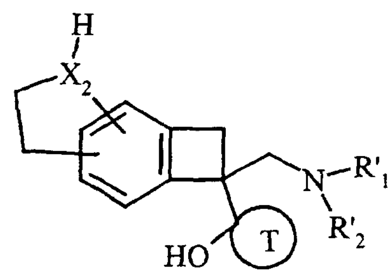

**(I/n)** **(I/o)**

dans laquelle T, $X_2$, $R'_1$ et $R'_2$ sont tels que définis précédemment,

**b) *soit*** un composé de formule **(II/1) :**

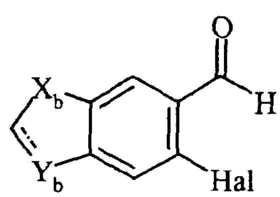

**(II/1)**

dans laquelle Hal représente un atome d'halogène, et $X_b$ représente un atome d'oxygène quand $Y_b$ représente un atome d'oxygène et ----- représente une liaison simple, ou $X_b$ représente un groupement -CH=CH- quand $Y_b$ représente un groupement CH et ----- représente une liaison double,
composés de formule (II/1) qui est mis à réagir avec $(EtO)_2POCH_2CN$ pour conduire aux composés de formule (II/2) :

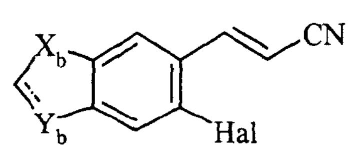

**(II/2)**

dans laquelle Hal, $X_b$ et $Y_b$ sont tels que définis précédemment,

composés de formule (II/2) qui est d'abord soumis à l'action d'un réducteur classique de la chimie organique puis qui est mis à réagir avec$NaNH_2$, pour conduire aux composés de formule (II/3) :

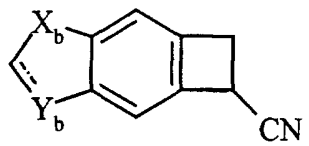

(II/3)

dans laquelle Xb et Yb sont tels que définis précédemment,
composés de formule (II/3) qui peuvent être traités dans les mêmes conditions que les composés de formule (V/a) par un composé de formule (VI), puis (VIIIa) ou (VIIIb), puis (VIIIc), pour conduire successivement aux composés de formules ($I/3_a$), ($I/3_b$) et ($I/3_c$) :

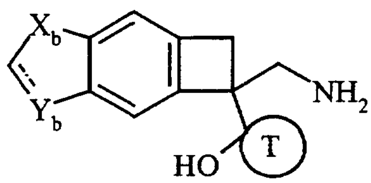

,

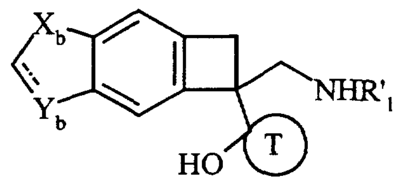

($I/3_a$) ($I/3_b$)

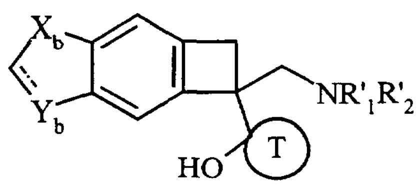

($I/3_c$)

dans lesquelles $X_b$, $Y_b$, T, $R'_1$ et $R'_2$ sont tels que définis précédemment,
les composés (I/a) à (I/o) et ($I/3_a$) à ($I/3_c$) forment l'ensemble des composés de l'invention, que l'on purifie le cas échéant, selon une technique classique de purification, qui peuvent, si on le désire, être séparés en leurs différents isomères selon une technique classique de séparation, et que l'on transforme, le cas échéant, en leurs sels d'addition à un acide ou à une base pharmaceutiquement acceptable.

Les composés de formule (II), (II/1), (III), (VI), (VIIIa), (VIIIb), (VIIIc), (IX), (XIII), (XV) et (XVI) sont soit des produits connus, soit des produits obtenus à partir de substances connues selon des procédés classiques de la chimie organique.

Les composés de la présente invention sont des inhibiteurs de la recapture de la sérotonine, de la noradrénaline et de la dopamine. Ils sont utiles, en tant que médicament, pour le traitement de la dépression, des attaques de panique, des troubles obsessionnels compulsifs, des phobies, des troubles impulsifs, de l'abus de drogue, de l'anxiété, de l'obésité et de la boulimie.

La présente invention a également pour objet les compositions pharmaceutiques contenant comme principe actif au moins un composé de formule (I), ses isomères optiques, ses sels d'addition à un acide ou à une base pharmaceutiquement acceptable, seul ou en combinaison avec un ou plusieurs excipients ou véhicules inertes, non toxiques, pharmaceutiquement acceptables.

Parmi les compositions pharmaceutiques selon l'invention, il sera cité plus particulièrement celles qui conviennent pour l'administration orale, parentérale (intraveineuse, intramusculaire ou sous-cutanée), per ou transcutanée, nasale, rectale, perlinguale, oculaire ou respiratoire, et notamment les comprimés simples ou dragéifiés, les comprimés sublinguaux, les gélules, les capsules, les suppositoires, les crèmes, les pommades, les gels dermiques, les préparations injectables ou buvables, les aérosols, les gouttes oculaires ou nasales, etc...

La posologie utile varie selon l'âge et le poids du patient, la voie d'administration, la nature et la sévérité de l'affection, et la prise de traitements éventuels associés et s'échelonne de 0,5 mg à 25 mg en une ou plusieurs prises par jour.

Les exemples suivants illustrent l'invention mais ne la limitent en aucune façon. Les produits de départ utilisés sont des produits connus ou préparés selon des modes opératoires connus. Les différentes préparations conduisent à des intermédiaires de synthèse utiles pour la préparation des composés de l'invention.

Les structures des composés décrits dans les exemples ont été déterminées selon les techniques spectrophotométriques usuelles (infrarouge, résonance magnétique nucléaire, spectrométrie de masse, ...).

Les points de fusion ont été déterminés soit à la platine chauffante de Kofler (K.), soit à la platine chauffante sous microscope (M.K.). Lorsque le composé existe sous forme de sel, le point de fusion donné correspond à celui du produit salifié.

A titre indicatif, la numérotation adoptée pour les différents systèmes tricycliques est la suivante :

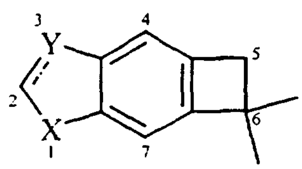 , 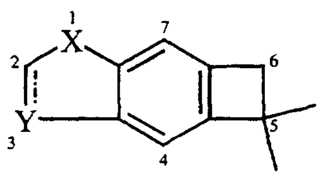 , 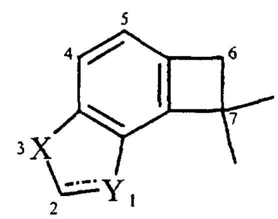

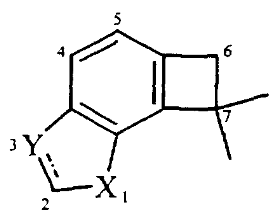 , 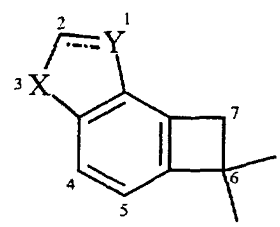 , 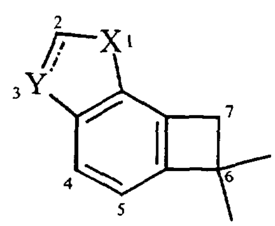

**<u>PREPARATION 1</u> :**

**6-Cyano-1-méthylsulfonyl 5,6-dihydrocyclobuta[*f*]indole**

***<u>Stade 1</u> : 1-Cyano-5-[(2,2-diméthoxyéthyl)amino]benzocyclobutane***

Sur une suspension de 13,5 g de 1-cyano-5-aminobenzocyclobutane dans 400 ml de 1,2-dichloroéthane sont ajoutés par un goutte à goutte rapide, 26,5 ml d'une solution de 2,2-diméthoxyacétaldéhyde à 45 % dans le terbutylméthyléther, puis 16 ml d'acide acétique et enfin, par fraction, 39,7 g de triacétoxyborohydrure de sodium. Après élévation de la température jusqu'à 29°C, on refroidit à température ambiante puis agite 1 h 15 et hydrolyse en versant sur 500 ml d'une solution aqueuse saturée en hydrogénocarbonate de sodium. La phase organique est séparée, lavée à l'eau et concentrée sous pression réduite pour conduire, avec un rendement quantitatif, au produit désiré.

***<u>Stade 2</u> :* 1-Cyano *5-[(N-(2,2-diméthoxyéthyl)N-méthylsulfonylamino] benzocyclobutane***

Une solution préparée à partir de 21,6 g du produit obtenu au stade 1 précédent, 58 ml de pyridine et 225 ml de dichlorométhane est refroidie à 0°C. 10,8 ml de chlorure de mésyle sont ajoutés au goutte à goutte pendant 20 minutes et on agite encore pendant 40 minutes à 0°C puis 20 heures à température ambiante. Le milieu réactionnel est ensuite versé sur 40 ml d'une solution aqueuse saturée en hydrogénocarbonate de sodium. Après décantation, la phase aqueuse est extraite par 2 fois 150 ml de chlorure de méthylène. Les phases organiques jointes sont lavées à l'acide chlorhydrique 1N, séchées et concentrées pour conduire au produit attendu avec un rendement quantitatif.

***<u>Stade 3</u> : 6-Cyano-1-méthylsulfonyl-5,6-dihydrocyclobuta[f]indole***

Sur 2,1 l de toluène à reflux sont coulés simultanément en 1 h 15, une solution de 10,9 ml de chlorure de

titane dans 450 ml de toluène et une solution de 27,9 g du produit obtenu au stade 2 dilué dans 450 ml de toluène. A la fin de l'addition, on laisse redescendre la température jusqu'à 40°C, puis verse sur 1,8 l d'une solution aqueuse saturée en hydrogénocarbonate de sodium. Après décantation, la phase aqueuse est extraite au toluène, les phases organiques sont réunies, lavées, séchées, concentrées. Le résidu est purifié par chromatographie sur gel de silice (dichlorométhane/cyclohexane : 75/25) pour conduire au produit attendu et à son régioisomère. **Point de fusion : 142-144°C (MK)**

**PREPARATION 2 :**

**6-Cyano-5,6-dihydrocyclobuta[*f*]indole**

2,6 g du produit de la préparation 1 sont introduits dans une solution de 7,7 g de potasse dans 190 ml de méthanol. Après 12 heures à reflux, le méthanol est évaporé et le résidu repris par de l'éther. Après lavage, la phase organique est séchée et concentrée pour conduire au produit attendu.
**Point de fusion : 126-128°C (MK)**

**PREPARATION 3 :**

**6-Cyano-2,3,5,6-tétrahydrocyclobuta[*f*]indole**

3,43 g du produit obtenu à la préparation 2 sont solubilisés dans 55 ml d'acide acétique. Dans le milieu réactionnel refroidi à 13°C, sont ajoutés, par portions, en 5 minutes, 3,84 g de cyanoborohydrure de sodium. Après retour à température ambiante, l'agitation est maintenue pendant 2 heures, puis le milieu réactionnel est refroidi à 0°C et amené à pH = 11 par addition d'une solution de soude (45 g dans 250 ml d'eau). La solution laiteuse obtenue est extraite à l'éther. Les phases organiques sont lavées, séchées et concentrées pour conduire au produit attendu sous forme de solide blanc.
**Point de fusion : 85-87°C (MK)**

**PREPARATION 4 :**

**4-Cyano-1-méthylsulfonyl-4,5-dihydrocyclobuta[*e*]indole**

Le régioisomère obtenu au stade 3 de la préparation 1 correspond au produit attendu.
**Point de fusion : 118-120°C (MK)**

**PREPARATION 5 :**

**4-Cyano-4,5-dihydrocyclobuta[*e*]indole**

Le produit est obtenu selon le procédé de la préparation 2, en utilisant comme substrat le produit de la préparation 4.
**Point de fusion : 132-134°C (MK)**

**PREPARATION 6 :**

**4-Cyano-2,3,4,5-tétrahydrocyclobuta[*e*]indole**

Le produit est obtenu selon le procédé de la préparation 3, en utilisant comme substrat le produit de la préparation 5.

**PREPARATION 7 :**

**5-Cyano-5,6-dihydrocyclobuta[*f*]benzothiophène**

***Stade 1 : 1-Cyano-4-thiométhylbenzocyclobutane***

Dans 3,5 l d'ammoniac liquide à reflux contenant des quantités catalytiques de ferrocyanure de potassium et de trinitrate de fer monohydrate, sont introduits en 2 heures 50,8 g de sodium, puis en 5 minutes, 153 g de 3-(2-chloro-5-thiométhylphényl) propionitrile. On laisse réagir 1 heure à -33°C puis on introduit 118,2 g de chlorure d'ammonium solide. Après évaporation de l'ammoniac, le résidu est repris par de l'éther, les sels sont filtrés et le filtrat est évaporé à sec permettant de conduire au produit attendu.

***Stade 2* : *1-Cyano-4-méthylsulfinylbenzocyclobutane***

11 g de produit du stade 1 précédent sont dissous dans 70 ml de dichlorométhane. Sur la solution refroidie à -5°C est coulée une solution de 15,8 g d'acide m-chloroperbenzoïque dans 80 ml de dichlorométhane. Après 10 minutes d'agitation, le milieu réactionnel est versé sur 100 ml d'eau et 100 ml de soude 1N. On décante, extrait à nouveau la phase aqueuse au dichlorométhane. Les phases organiques réunies sont lavées à l'hydrogénosulfate de sodium, puis à la soude 1N, au bicarbonate de sodium et enfin à l'eau jusqu'à neutralité. La phase organique est séchée et concentrée pour conduire au produit attendu.

***Stade 3* : *1-Cyano-4-mercaptobenzocyclobutane***

Sur une solution de 11 g de produit obtenu au stade précédent dans 100 ml de dichlorométhane sont ajoutés 9,76 ml d'acide trifluoroacétique dissous dans 20 ml de dichlorométhane. L'addition dure 45 minutes durant laquelle la température est maintenue à 25°C. Le milieu réactionnel est encore agité 1 h 30 à cette température, puis évaporé à sec. Le résidu est repris 10 minutes sous agitation en présence d'un mélange 50/50 de triéthylamine et de méthanol. Après évaporation, on dilue au dichlorométhane, lave par une solution saturée de chlorure d'ammonium puis à l'eau, sèche et concentre pour obtenir le produit attendu sous forme d'une huile.

***Stade 4* : *1-Cyano-4-(2,2-diéthoxyéthylsulfanyl)benzocyclobutane***

Sur une solution de 9,1 g du produit obtenu au stade 3 précédent dans 20 ml de tétrahydrofurane et 140 ml d'éthanol sont additionnés, à 20°C et par portions 3,65 g de borohydrure de sodium. Après 1 heure à 50°C, 2,14 g de borohydrure de sodium solide sont à nouveau ajoutés. Le milieu réactionnel est ensuite porté à 80°C et à cette température on coule en 1 heure, 39 ml de diéthylacétal du bromoacétaldéhyde. Le chauffage est encore maintenu pendant 12 heures, puis le milieu réactionnel refroidi est versé sur 1 litre d'eau glacée. Le mélange obtenu est extrait à l'éther et la phase organique est lavée avec une solution de bicarbonate de sodium à 10 % puis à l'eau. La phase étherée est séchée et concentrée sous vide pour conduire au produit attendu sous forme d'huile.

***Stade 5* : 5-*Cyano-5,6-dihydrocyclobuta[f]benzothiophène***

10 g d'acide polyphosphorique, 8,5 g du produit obtenu au stade 4 précédent dans 500 ml de chlorobenzène sont portés à 130°C pendant 4 heures. Après refroidissement, le surnageant est soutiré et le résidu est rincé au dichlorométhane. Le surnageant et la phase dichlorométhane sont réunies et neutralisées en ajoutant du bicarbonate de sodium en poudre. Après 15 minutes d'agitation, on filtre et évapore à sec. Le résidu est purifié par chromatographie sur gel de silice (cyclohexane/dichlorométhane : 50/50) permettant d'obtenir le produit attendu ainsi qu'un produit secondaire.
**Point de fusion : 102-103°C (MK)**

**PREPARATION 8 :**

**6-Cyano-5,6-dihydrocyclobuta[*f*]benzothiophène**

Le produit est obtenu selon le procédé de la préparation 7 des stades 1 à 5 mais en utilisant au stade 1 le 3-(3-bromo-4-thiométhylphényl)propionitrile à la place du 3-(2-chloro-5-thiométhylphényl)propionitrile.

**PREPARATION 9 :**

**5-Cyano-5,6-dihydrocyclobuta[*f*]benzofurane**

***Stade 1* : *1-Cyano-4-(2,2-diéthoxyéthoxy)benzocyclobutane***

Une solution de 16,5 g de 1 -cyano-4-hydroxybenzocyclobutane dans 205 ml de diméthylformamide est coulée sur une suspension de 67,8 mmol d'hydrure de sodium dans 160 ml de diméthylformamide. Le milieu réactionnel est agité 30 minutes puis une solution de 10,2 ml de 1-bromo-2,2-diéthoxyéthane dans 40 ml est additionnée en 15 minutes

et la température est ensuite portée à 60°C pendant 6 heures. On laisse encore 12 heures sous agitation, évapore le diméthylformamide, reprend à l'eau, extrait au dichlorométhane, sèche et concentre sous vide. Le résidu est chromatographié sur gel de silice ($CH_2Cl_2$/AcOEt : 95/5) permettant d'isoler le produit attendu.

***Stade 2* : 5-Cyano-5,6-dihydrocyclobuta[f]benzofurane**

Le produit est obtenu comme au stade 3 de la préparation 1 mais en utilisant le produit du stade 1 précédent. Au cours d'une chromatographie sur gel de silice ($CH_2Cl_2$/cyclohexane : 80/20), on isole le produit attendu ainsi qu'un produit secondaire.

**PREPARATION 10 :**

**6-Cyano-5,6-dihydrocyclobuta[*f*]benzofurane**

Le produit est obtenu comme le produit de la préparation 9 mais en utilisant au stade 1 le 1-cyano-5-hydroxybenzocyclobutane et au stade 2 comme éluant dans la séparation des régioisomères le mélange $CH_2Cl_2$/cyclohexane : 75/25. Le produit attendu est isolé sous forme d'une huile.

**PREPARATION 11 :**

**5-Cyano-4,5-dihydrocyclobuta[*e*]benzothiophène**

Le produit secondaire obtenu au stade 5 de la préparation 7 correspond au produit attendu.

**PREPARATION 12 :**

**4-Cyano-4,5-dihydrocyclobuta[*e*]benzothiophène**

Le produit est obtenu au cours du stade 5 de la préparation 8.

**PREPARATION 13 :**

**5-Cyano-4,5-dihydrocyclobuta[*e*]benzothiophène**

Le produit secondaire obtenu au stade 2 de la préparation 9 correspond au produit attendu.

**PREPARATION 14 :**

**4-Cyano-4,5-dihydrocyclobuta[*e*]benzofurane**

Le produit secondaire isolé lors de la chromatographie de la préparation 10 correspond au produit attendu.

**PREPARATION 15 :**

**5,6-Dihydrocyclobuta[*f*][1,3]benzodioxole-5-carbonitrile**

46 g de 3-(6-bromo-1,3-benzodioxol-5-yl)propanenitrile sont introduits par portions dans 1,4 l d'ammoniac liquide dans lequel est dissous de l'amidure de sodium préalablement préparé par introduction de 16,7 g de sodium dans l'ammoniac liquide. Après 30 minutes de contact, le milieu réactionnel est traité par 38,7 g de chlorure d'ammonium puis l'ammoniac est distillé à température ambiante. Le résidu est repris par l'éther et filtré ; le précipité est lavé à l'éther. Les phases éthérées réunies sont évaporées et le résidu obtenu est recristallisé de l'alcool isopropylique permettant d'isoler le produit attendu.
**Point de fusion : 91°C (M.K.)**

**PREPARATION 16 :**

**6,7-Dihydrocyclobuta[*g*][1]benzofuran-7-carbonitrile**

***Stade 1* : *1-Cyano-6-(2,2-diéthoxyéthoxy) benzocyclobutane***

Le produit est obtenu selon le procédé du stade 1 de la préparation 9 en utilisant comme substrat le 1-cyano-6-hydroxybenzocyclobutane.

***Stade 2* : *6,7-Dihydrocyclobuta[g][1]benzofuran-7-carbonitrile***

Le produit est obtenu selon le procédé du stade 3 de la préparation 1 en utilisant comme substrat le produit obtenu au stade 1 précédent.

**PREPARATION 17 :**

**1,2-Dihydrocyclobuta[*b*]naphthalène-1-carbonitrile**

Le produit est obtenu selon le procédé de la préparation 15 en utilisant comme substrat le 3-(3-iodo-2-naphthyl)propionitrile.
**Point de fusion : 98-102°C (M.K.)**

**PREPARATION 18 :**

**1,2-Dihydrocyclobuta[*a*]naphthalène-1-carbonitrile**

***Stade 1* : *1,1-Diéthoxy-1,2-dihydrocyclobuta[a]naphthalène***

9,4 g d'amidure de sodium sont introduits dans 350 ml de tétrahydrofurane. Sur ce mélange sont coulés successivement 25,9 g de 1-bromonaphtalène et 28 g de 1,1-diéthoxyethylène fraîchement préparé. Le milieu réactionnel est porté à reflux pendant 16 heures puis repris à l'eau et l'éther. Après lavage à pH neutre de la phase organique et séchage de celle-ci, le produit attendu est isolé et chromatographié sur gel de silice (dichlorométhane/cyclohexane : 1/1).

***Stade 2* : *Cyclobuta[*a*]naphthalèn-1(2H)-one***

10g du produit obtenu au stade 1 sont traités à température ambiante par 42 ml d'acide chlorhydrique 1N dissous dans 170 ml de tétrahydrofurane. Après 1h30 de contact, le solvant est concentré et le résidu est versé sur 170 ml d'eau. 6,9 g d'un solide correspondant au produit attendu sont alors isolé.
**Pont de fusion : 92-94°C (M.K)**

***Stade 3* : *1,2-Dihydrocyclobuta[*a*]naphthalèn-1-ol***

1,4 g de borohydrure de sodium sont ajoutés à une suspension, à 0-5°C, de 5,2 g de produit obtenu au stade 2 dans 150 ml de méthanol. Après 15 minutes à cette température puis 1 heure à température ambiante, le milieu réactionnel est coulé sur 300 g de glace et extrait au dichlorométhane. Après traitement usuel, 5,2 g du produit attendu sont isolés.
**Point de fusion : 96-100°C (M.K.)**

***Stade 4* : *1,2-Dihydrocyclobuta[*a*]naphthalène-1-carbonitrile***

5,1 g du produit obtenu au stade 3, 14,2 g de triphénylphosphine, 12 g de tétrabromure de carbone dans 150 ml d'éther sont portés 2 heures à reflux. Après refroidissement, le milieu réactionnel est filtré, concentré, repris par 50 ml d'éther, filtré et évaporé. Les 7,85 g du résidu obtenu sont traités par 10,5 g de tétrabutylammonium cyanure dans 150 ml de tétrahydrofurane. Après 64 heures de contact à température ambiante, on concentre le milieu réactionnel et on le reprend par de la glace et de l'éther. Après décantation, une huile est isolée qui est purifiée par chromatographie sur gel de silice (dichlorométhane) permettant d'isoler le produit attendu.
**Point de fusion : 78-84°C**

**PREPARATION 19 :**

**1-(Méthylsulfonyl)-5,6-dihydro-1*H*-cyclobuta[*f*]indole-5-carbonitrile**

Le produit est obtenu selon le procédé de la préparation 1 des stades 1 à 3 en utilisant au stade 1 le 4-amino-1-cyanobenzocyclobutane.
**Point de fusion : 164-168°C (M.K.)**

**PREPARATION 20 :**

**5,6-Dihydro-1*H*-cyclobuta[*f*]indole-5-carbonitrile**

Le produit est obtenu selon le procédé de la préparation 2 mais en utilisant comme substrat le produit de la préparation 19.
**Point de fusion : 109-113°C (M.K.)**

**PREPARATION 21 :**

**2,3,5,6-Tetrahydro-1*H*-cyclobuta[*f*]indole-5-carbonitrile**

Le produit est obtenu selon le procédé de la préparation 3 mais en utilisant comme substrat le produit de la préparation 20.
**Point de fusion : 100-105°C (M.K.)**

**EXEMPLE 1 :**

**1-[6-(Aminométhyl)-2,3,5,6-tétrahydro-*1H*-cyclobuta[*f*]indol-6-yl]cyclohexanol**

***Stade A : 6-(1-Hydroxycyclohexyl)-2,3,5,6-tétrahydro-1H-cyclobuta[f]indole-6-carbonitrile***

4,1 g du produit obtenu à la préparation 3 sont dissous dans 215 ml de tétrahydrofurane. Le milieu réactionnel est refroidi à -80°C et 19,25 ml d'une solution 2,5 M de n-butyllithium dans l'hexane sont additionnés. A la fin de l'addition, l'agitation est maintenue 20 minutes puis 6,2 ml de cyclohexanone sont coulés en 3 minutes. Après 2 heures de contact à 80°C, on laisse revenir à température ambiante et introduit 23 ml d'une solution aqueuse saturée de chlorure d'ammonium, puis 135 ml d'eau. Après décantation, la phase organique est lavée par une solution saturée de chlorure de sodium, séchée, concentrée. Le résidu obtenu est concrétisé de l'éther isopropylique, filtré pour obtenir le produit désiré et le filtrat est purifié par chromatographie sur gel de silice ($CH_2Cl_2$/AcOEt : 90/10) pour isoler une quantité supplémentaire de produit attendu.
**Point de fusion : 168-170°C**

***Stade B : 1-[6-(Aminométhyl)-2,3,5,6-tétrahydro-1H-cyclobuta[f]indol-6-yl] cyclohexanol***

4 g du produit obtenu au stade A précédent sont dissous dans 200 ml d'une solution 3,6 N de méthanol ammoniacal contenant 2 ml de nickel de Raney. Le mélange réactionnel est hydrogéné sous une pression de 30 bars à 60°C pendant 24 heures. Après filtration et évaporation du solvant, le résidu est repris par du dichlorométhane, lavé à l'eau jusqu'à neutralité, séché et concentré pour isoler le produit attendu sous forme d'une huile.

**EXEMPLE 2 :**

**1-{6-[(Diméthylamino)méthyl]-1-méthyl-2,3,5,6-tétrahydro-1*H*-cyclobuta[*f*]indol-6-yl}cyclohexanol**

659 mg du produit de l'exemple 1 sont dissous dans 20 ml d'acétonitrile. Dans la solution refroidie à 0°C, 608 mg de cyanoborohydrure de sodium et 1,5 ml d'une solution de formaldéhyde à 37 % dans l'eau sont introduits en maintenant la température à 0°C. Après 20 heures à température ambiante, on hydrolyse par 33 ml d'acide chlorhydrique 1 N, et agité pendant 3 heures. Le mélange réactionnel est lavé avec 30 ml d'éther puis basifié par de la soude à 20 %. La phase aqueuse est extraite au dichlorométhane. Après séchage et évaporation, le résidu est purifié par chromatographie sur gel de silice ($CH_2Cl_2$/EtOH : 95/5) pour conduire au produit attendu.
**Point de fusion : 121-124°C (MK)**

**<u>EXEMPLE 3</u> :**

**1-{6-[(Diméthylamino)méthyl]-2,3,5,6-tétrahydro-1*H*-cyclobuta[*f*]indol-6-yl} cyclohexanol**

***<u>Stade A</u> : 6-(1-Hydroxycyclohexyl)-1-(méthylsulfonyl)-2,3,5,6-tétrahydro-1H-cyclobuta[f]indole-6-carbonitrile***

On procède comme au stade 2 de la préparation 1 mais en utilisant le produit du stade A de l'exemple 1. Le produit attendu est concrétisé dans l'éther sous forme d'un solide violet.
**<u>Point de fusion</u> : 174-176°C**

***<u>Stade B</u> : 1-[6-(Aminométhyl)-1-(méthylsulfonyl)-2,3,5,6-tétrahydro-1H-cyclobuta[f]indol-6-yl]cyclohexanol***

On procède comme au stade B de l'exemple 1 mais en utilisant le produit du stade A ci-dessus. On obtient le produit attendu sous forme d'une meringue jaune.

***<u>Stade C</u> : 1-[6-[(Diméthylamino)méthyl]-1-(méthylsulfonyl)-2,3,5,6-tétrahydro-1H-cyclobuta[f]indol-6-yl]cyclohexanol***

On procède dans l'exemple 2 mais en utilisant le produit du stade B ci-dessus. Le produit attendu est obtenu sous forme d'un solide jaune.
**<u>Point de fusion</u> : 156-158°C (MK)**

***<u>Stade D</u> : 1-{6-[(Diméthylamino)méthyl]-2,3,5,6-tétrahydro-1H-cyclobuta[f]indol-6-yl}cyclohexanol***

40 ml d'ammoniac liquide sont introduits dans un tricol puis 380 mg du produit obtenu au stade C dissout dans 10 ml de tétrahydrofurane. On refroidit à -50°C et introduit dans le milieu réactionnel 100 mg de sodium en plusieurs fois. Après 15 minutes de contact, on introduit par fractions 430 mg de chlorure d'ammonium en poudre. On laisse ensuite remonter à température ambiante. Après évaporation de tout l'ammoniac, on reprend à l'eau, extrait à l'éther, sèche et concentre, pour obtenir le produit attendu.
**<u>Point de fusion</u> : 159-161°C (MK)**

**<u>EXEMPLE 4</u> :**

**1-[5-(Aminométhyl)-5,6-dihydrocyclobuta[*f*][1]benzofuran-5-yl]cyclohexanol**

***<u>Stade A</u> : 5-(1-Hydroxycyclohexyl)-5,6-dihydrocyclobuta[f][1]benzofuran-5-carbonitrile***

On procède comme au stade A de l'exemple 1 mais en utilisant le produit de la préparation 9 et en effectuant l'hydrolyse du milieu réactionnel à -75°C. On isole le produit attendu sous forme d'un solide.
**<u>Point de fusion</u> : 154-158°C (MK)**

***<u>Stade B</u> : 1-[5-(Aminométhyl)-5,6-dihydrocyclobuta[f][1]benzofuran-5-yl] cyclohexanol***

Une solution de 520 mg du produit obtenu au stade A précédent dans 10 ml de tétrahydrofurane est additionnée goutte à goutte à 0°C, sous courant d'azote, à une suspension de 177 mg d'aluminohydrure de sodium dans 10 ml d'éther. Le milieu réactionnel est agité une heure à température ambiante puis hydrolysé par 0,26 ml d'eau, 0,79 ml de soude à 20 % et 1,05 ml d'eau. Après 15 minutes d'agitation à température ambiante, on filtre et évapore à sec. Le produit attendu est obtenu sous forme d'une meringue collante.

**<u>EXAMPLE 5</u> :**

**1-{5-[(Diméthylamino)méthyl]-5,6-dihydrocyclobuta[*f*][1]benzofuran-5-yl} cyclohexanol**

400 mg du produit de l'exemple 4 sont solubilisés dans 12 ml d'acétonitrile. Après refroidissement à 0°C sont successivement ajoutés 0,55 ml d'une solution de formaldéhyde à 37 % dans l'eau et 185 mg de cyanoborohydrure de sodium . Après 1 heure à 0°C et 2 heures à température ambiante, 0,55 ml de la solution de formaldéhyde à 37 % dans l'eau et 185 mg de cyanoborohydrure de sodium sont à nouveau ajoutés et l'agitation est encore maintenue 12 heures à 20°C. On hydrolyse à température ambiante par 22,3 ml d'acide chlorhydrique (1N) et agite 1 heure, basifie

à 0°C par de la soude à 20 %, extrait au dichlorométhane, lave à l'eau, sèche et concentre. Une chromatographie sur gel de silice ($CH_2Cl_2$/EtOH : 90/10) permet d'isoler le produit attendu sous forme de cristaux blancs.
**Point de fusion : 127-136°C (M.K)**

**EXEMPLE 6 :**

**1-[5-(Aminométhyl)-5,6-dihydrocyclobuta[*f*][1]benzothien-5-yl]cyclohexanol**

On procède comme dans l'exemple 4, des stades A à B, en utilisant au stade A le produit de la préparation 7.

**EXEMPLE 7 :**

**1-{5-[(Diméthylamino)méthyl]-5,6-dihydrocyclobuta[*f*][1]benzothien-5-yl} cyclohexanol**

On procède comme dans l'exemple 5 en utilisant comme substrat le produit de l'exemple 6.
**Point de fusion : 151-155°C (MK)**

**EXEMPLE 8 :**

**1-{6-[(Diméthylamino)méthyl]-1-méthyl-5,6-dihydro-1*H*-cyclobuta[*f*]indol-6-yl}** cyclohexanol

0,44 g du produit de l'exemple 1 et 1,23 g de dioxyde de manganèse sont introduits dans 15 ml de toluène. Le milieu réactionnel est agité 24 heures à température ambiante puis 0,6 g de dioxyde de manganèse sont à nouveau introduits, l'agitation étant poursuivie 24 heures supplémentaires. Après filtration sur célite et évaporation du solvant, le résidu est purifié par chromatographie rapide sur gel de silice ($CH_2Cl_2$/MeOH/$NH_4OH$ : 98/20/0,2 %) pour conduire au produit attendu.
**Point de fusion : 148-150°C (MK)**

**EXEMPLE 9 :**

**1-{7-[(Diméthylamino)méthyl]-2,3,6,7-tétrahydro-1*H*-cyclobuta[e]indol-7-yl} cyclohexanol**

***Stade A : 7-(1-Hydroxycyclohexyl)-2,3,6,7-tétrahydro-1H-cyclobuta[e]indole-7-carbonitrile***

Le produit est obtenu comme au stade A de l'exemple 1 mais en utilisant le produit de la préparation 6 au lieu du produit de la préparation 3.

***Stade B : 1-{7-[(Diméthylamino)méthyl]-2,3,6,7-tétrahydro-1H-cyclobuta[e]indol-7-yl}cyclohexanol***

On procède comme à l'exemple 3 des stades A à D mais en utilisant au stade A de cet exemple le produit du stade A ci-dessus.

**EXEMPLE 10 :**

**1-[6-(Aminométhyl)-5,6-dihydrocyclobuta[*f*][1]benzothien-6-yl]cyclohexanol**

Le produit est obtenu selon le procédé de l'exemple 4 des stades A à B, en utilisant comme substrat au stade A le composé de la préparation 8.

**EXEMPLE 11 :**

**1-[6-(Aminométhyl)-5,6-dihydrocyclobuta[*f*][1]benzofuran-6-yl]cyclohexanol**

Le produit est obtenu selon le procédé de l'exemple 4 des stades A à B, en utilisant comme substrat au stade A le composé de la préparation 10.

**EXEMPLE 12 :**

**1-{6-[(Diméthylamino)méthyl]-5,6-dihydrocyclobuta[*f*][1]benzofuran-6-yl} cyclohexanol**

Le produit est obtenu selon le procédé de l'exemple 5, en utilisant comme substrat le composé de l'exemple 11.

**EXEMPLE 13 :**

**1-[6-(Aminométhyl)-6,7-dihydrocyclobuta[*e*][1]benzothien-6-yl]cyclohexanol**

Le produit est obtenu selon le procédé de l'exemple 4 des stades A à B, en utilisant comme substrat au stade A le composé de la préparation 11.

**EXEMPLE 14 :**

**1-[7-(Aminométhyl)-6,7-dihydrocyclobuta[*e*][1]benzothien-7-yl]cyclohexanol**

Le produit est obtenu selon le procédé de l'exemple 4 des stades A à B, en utilisant comme substrat au stade A le composé de la préparation 12.

**EXEMPLE 15 :**

**1-[6-(Aminométhyl)-6,7-dihydrocyclobuta[*e*][1]benzofuran-6-yl]cyclohexanol**

Le produit est obtenu selon le procédé de l'exemple 4 des stades A à B, en utilisant comme substrat au stade A le composé de la préparation 13.

**EXEMPLE 16 :**

**1-[7-(Aminométhyl)-6,7-dihydrocyclobuta[*e*][1]benzofuran-7-yl]cyclohexanol**

Le produit est obtenu selon le procédé de l'exemple 4 des stades A à B, en utilisant comme substrat au stade A le composé de la préparation 14.

**EXEMPLE 17 :**

**1-{6-[(Diméthylamino)méthyl]-5,6-dihydrocyclobuta[*f*][1]benzothièn-6-yl} cyclohexanol**

Le produit est obtenu selon le procédé de l'exemple 2, en utilisant comme substrat le composé de l'exemple 10.
**Point de fusion : 161-166°C (M.K.)**

**EXEMPLE 18 :**

**1-{5-[(Diméthylamino)méthyl]-5,6-dihydrocyclobuta[*f*][1,3]benzodioxol-5-yl}cyclohexanol**

Le produit est obtenu selon le procédé de l'exemple 1 des stades A à B, puis selon le procédé de l'exemple 2, en utilisant comme substrat au stade A de l'exemple 1 le produit de la préparation 15 à la place de celui de la préparation 3.
**Point de fusion : 94-96°C (M.K.)**

**EXEMPLE 19 :**

**1-{5-[(Diméthylamino)méthyl]-5,6-dihydrocyclobuta[*f*][1]benzofuran-5-yl}cyclopentanol et son chlorhydrate**

Le produit est obtenu selon le procédé de l'exemple 4 des stades A à B, puis selon le procédé de l'exemple 5, en utilisant comme substrat au stade A la cyclopentanone à la place de la cyclohexanone. La base libre est transformée en son chlorhydrate par action de l'éther chlorhydrique.
**Point de fusion : 258-262°C (M.K.)**

**EXEMPLE 20 :**

**1-{5-[(Diméthylamino)méthyl]-5,6-dihydrocyclobuta[*f*][1,3]benzodioxol-5-yl} cyclopentanol**

Le produit est obtenu selon le procédé de l'exemple 4 des stades A à B, puis selon le procédé de l'exemple 5, en utilisant comme substrat au stade A la cyclopentanone à la place de la cyclohexanone et le produit de la préparation 15 à la place de celui de la préparation 9.
**Point de fusion : 71-74°C (M.K.)**

**EXEMPLE 21 :**

**1-[5-(Aminométhyl)-5,6-dihydrocyclobuta[*f*][1]benzothièn-5-yl]cyclopentanol**

Le produit est obtenu selon le procédé de l'exemple 4 des stades A à B en utilisant comme substrat au stade A la cyclopentanone à la place de la cyclohexanone et le produit de la préparation 7 à la place de celui de la préparation 9.
**Point de fusion : 149-153°C (M.K.)**

**EXEMPLE 22 :**

**1-{5-[(Méthylamino)méthyl]-5,6-dihydrocyclobuta[*f*][1]benzothièn-5-yl}cyclopentanol**

Sur un mélange de 1 g du produit de l'exemple 21, 1,2 ml de triéthylamine et 60 ml de dichlorométhane est introduite, à 0°C, une solution de 0,29 ml de chloroformiate d'éthyle dans 40 ml de dichlorométhane. Après retour à température ambiante, le milieu réactionnel est lavé par de l'acide chlorhydrique 0,1 N puis par une solution saturée de bicarbonate de sodium, séché puis évaporé. Le résidu obtenu est dissout dans 20 ml de tétrahydrofurane. La solution est ajoutée à un mélange de 1,3 g de $AlLiH_4$ dans 60 ml de tétrahydrofurane. Le milieu réactionnel est porté au reflux pendant 3 heures puis hydrolysé par 0,65 ml d'eau, 0,45 ml de soude à 20% et 2,1 ml d'eau. Après filtration et concentration, le résidu est purifié par chromatographie sur gel de silice (dichlorométhane/éthanol/$NH_4OH$ : 95/5/0,5) et recristallisé de l'éther isopropylique pour conduire au produit attendu.
**Point de fusion : 166-169°C (M.K.)**

**EXEMPLE 23 :**

**1-{5-[(Diméthylamino)méthyl]-5,6-dihydrocyclobuta[*f*][1]benzothièn-5-yl} cyclopentanol**

Le produit est obtenu selon le procédé de l'exemple 5 en utilisant le produit de l'exemple 21 à la place de celui de l'exemple 4.
**Point de fusion : 93-98°C (M.K.)**

**EXEMPLE 24 :**

**(+) 1-{5-[(Diméthylamino)méthyl]-5,6-dihydrocyclobuta[*f*][1]benzothièn-5-yl} cyclopentanol**

Le produit de l'exemple 23 est injectée sur une colonne chirale avec une phase mobile composé de méthanol/diéthylamine : 1000/1. Le premier produit élué correspond à l'isomère (+).
**Point de fusion : 122-126°C (M.K.)**

**EXEMPLE 25 :**

**(-) 1-{5-[(Diméthylamino)méthyl]-5,6-dihydrocyclobuta[*f*][1]benzothièn-5-yl}** cyclopentanol

Le produit de l'exemple 23 est injectée sur une colonne chirale avec une phase mobile composé de méthanol/diéthylamine : 1000/1. Le deuxième produit élué correspond à l'isomère (-).
**Point de fusion : 123-127°C (M.K.)**

**EXEMPLE 26 :**

**1-[6-(Aminométhyl)-5,6-dihydrocyclobuta[*f*][1]benzothièn-6-yl]cyclopentanol**

Le produit est obtenu selon le procédé de l'exemple 4 des stades A à B en utilisant comme substrat au stade A la cyclopentanone à la place de la cyclohexanone et le produit de la préparation 8 à la place de celui de la préparation 9.
**Point de fusion : 141-143°C (M.K.)**

**EXEMPLE 27 :**

**1-{6-[(Méthylamino)méthyl]-5,6-dihydrocyclobuta[*f*][1]benzothièn-6-yl}cyclopentanol**

Le produit est obtenu selon le procédé de l'exemple 22 en utilisant le produit de l'exemple 26 à la place de celui de l'exemple 21.
**Point de fusion : 117-120°C (M.K.)**

**EXEMPLE 28 :**

**1-{6-[(Diméthylamino)méthyl]-5,6-dihydrocyclobuta[*f*][1]benzothièn-6-yl}cyclopentanol**

Le produit est obtenu selon le procédé de l'exemple 5 en utilisant le produit de l'exemple 26 à la place de celui de l'exemple 4.
**Point de fusion : 94-96°C (M.K.)**

**EXEMPLE 29 :**

**1-{6-[(Diméthylamino)méthyl]-1-méthyl-5,6-dihydro-1*H*-cyclobuta[*f*]indol-6-yl} cyclopentanol**

Le produit est obtenu successivement selon le procédé des exemples 1, 2 et 8, en utilisant au stade A de l'exemple 1 la cyclopentanone à la place de la cyclohexanone.
**Point de fusion : 108-111°C (M.K.)**

**EXEMPLE 30 :**

**1-{5-[(diméthylamino)méthyl]-1-méthyl-5,6-dihydro-1*H*-cyclobuta[*f*]indol-5-yl} cyclopentanol**

Le produit est obtenu successivement selon le procédé des exemples 1, 2 et 8, en utilisant au stade A de l'exemple 1 la cyclopentanone à la place de la cyclohexanone et le produit de la préparation 21 à la place de celui de la préparation 3.
**Point de fusion : 100-104°C (M.K.)**

**EXEMPLE 31 :**

**1-{7-[(Diméthylamino)méthyl]-6,7-dihydrocyclobuta[*g*][1]benzofuran-7-yl} cyclopentanol**

Le produit est obtenu selon le procédé de l'exemple 4 des stades A à B, puis selon le procédé de l'exemple 5, en utilisant comme substrat au stade A la cyclopentanone à la place de la cyclohexanone et le produit de la préparation 16 à la place de celui de la préparation 9.
**Point de fusion : 135-137°C (M.K.)**

**EXEMPLE 32 :**

**1-{6-[(Diméthylamino)méthyl]-5,6-dihydrocyclobuta[*f*][1]benzofuran-6-yl} cyclopentanol et son chlorhydrate**

Le produit est obtenu selon le procédé de l'exemple 4 des stades A à B, puis selon le procédé de l'exemple 5, en utilisant comme substrat au stade A la cyclopentanone à la place de la cyclohexanone et le produit de la préparation 10 à la place de celui de la préparation 9.Le chlorhydrate est préparé par action de l'éther chlorhydrique.

**Point de fusion (chlorhydrate) : 225-235°C (M.K.)**

**EXEMPLE 33 :**

**1-{6-[(Diméthylamino)méthyl]-2,3,5,6-tétrahydro-1*H*-cyclobuta[*f*]indol-6-yl} cyclopentanol**

Le produit est obtenu selon le procédé de l'exemple 3 des stades A à B en utilisant au stade A le produit préparé au stade A de l'exemplel à partir de la cyclopentanone et non pas de la cyclohexanone.
**Point de fusion : 105-108°C (M.K.)**

**EXEMPLE 34 :**

**1-{6-[(Diméthylamino)méthyl]-5,6-dihydro-1*H*-cyclobuta[*f*]indol-6-yl}cyclopentanol**

Le produit est obtenu selon le procédé de l'exemple 8 en utilisant comme substrat le produit de l'exemple 33.
**Point de fusion : 180-184°C (M.K.)**

**EXEMPLE 35 :**

**1-{1-[(Diméthylamino)méthyl]-1,2-dihydrocyclobuta[*b*]naphthalèn-1-yl}cyclopentanol**

Le produit est obtenu selon le procédé de l'exemple 4 des stades A à B, puis selon le procédé de l'exemple 5, en utilisant comme substrat au stade A la cyclopentanone à la place de la cyclohexanone et le produit de la préparation 17 à la place de celui de la préparation 9.
**Point de fusion : 133-135°C (M.K.)**

**EXEMPLE 36 :**

**1-{7-[(Diméthylamino)méthyl]-6,7-dihydro-3*H*-cyclobuta[*e*]indol-7-yl}cyclopentanol**

Le produit est obtenu selon le procédé de l'exemple 3 des stades A à D, puis de l'exemple 8 en utilisant au stade A de l'exemple 3 le produit préparé au stade A de l'exemple 1 mais en utilisant d'une part le produit de la préparation 9 et d'autre part la cyclopentanone.
**Point de fusion : 200-204°C (M.K.)**

**EXEMPLE 37 :**

**1-{1-[(Dimethylamino)methyl]-1,2-dihydrocyclobuta[*a*]naphthalèn-1-yl}cyclopentanol et son chlorhydrate**

Le produit est obtenu selon le procédé de l'exemple 4 des stades A à B, puis selon le procédé de l'exemple 5, en utilisant comme substrat au stade A la cyclopentanone à la place de la cyclohexanone et le produit de la préparation 18 à la place de celui de la préparation 9. Le chlorhydrate est préparé à partir de l'éther chlorhydrique.
**Point de fusion (chlorhydrate) : 258-262°C (M.K.)**

**EXEMPLE 38 :**

**1-(7-{[((2S) 2,3-Dihydro-1,4-benzodioxin-2-ylméthyl)amino]méthyl}-6,7-dihydrocyclo-buta[*g*][1]benzofuran-7-yl)cyclopentanol et son chlorhydrate**

***Stade A* : 1-[7-(Aminométhyl)-6,7-dihydrocyclobuta[g][1]benzofuran-7-yl] cyclopentanol**

Le produit est obtenu selon le procédé de l'exemple 4 des stades A à B en utilisant comme substrat au stade A la cyclopentanone à la place de la cyclohexanone et le produit de la préparation 16 à la place de celui de la préparation 9.

***Stade B** : (2R)* **N-**{*[7-(1-Hydroxycyclopentyl)-6,7-dihydrocyclobuta[g][1]benzofuran-7-yl]methyl*}*-2,3-dihydro-1,4-benzodioxine-2-carboxamide*

Sur 900 mg du composé obtebu au stade A dans 30 ml de dichlorométhane et 1,2 ml de diisopropyléthylamine, à 0°C, est ajoutée une solution de 762 mg de chlorure de l'acide (2R) 2,3-dihydrobenzo[1,4]dioxin-2-yl carboxylique. Après 48 heures à température ambiante, le milieu réactionnel est dilué à l'eau, extrait au dichlorométhane. Un traitement classique des phases organiques permet après évaporation sous pression réduite d'isoler le produit attendu sous forme de meringue.

***Stade C** : 1-(7-{[((2S) 2,3-Dihydro-1,4-benzodioxin -2 -ylméthyl)amino]méthyl}-6,7-dihydrocyclobuta[g][1]benzofuran-7-yl)cyclopentanol et son chlorhydrate*

500 mg d'hydrure de lithium aluminium dans 20 ml de tétrahydrofurane sont portés à reflux, puis 1,1 g du produit obtenu au stade B dissout dans 20 ml de tétrahydrofurane sont ajoutés. Après 3h30 de relux, le milieu réactionnel est hydrolysé par addition de 0,45 ml d'eau, 0,31 ml de soude à 20% et 1,67 ml d'eau. Après filtration et évaporation, une chromatographie sur gel de silice du résidu (dichlorométhane/éthanol : 98/2) permet d'isoler le produit attendu qui est transformé en son chlorhydrate par action de l'éther chlorhydrique.
**Point de fusion (chlorhydrate) : 213-235°C**

**EXEMPLE 39 :**

**1-(5-{[((2R) 2,3-Dihydro-1,4-benzodioxin-2-ylméthyl)amino]méthyl}-5,6-dihydrocyclobuta[*f*][1,3]benzodioxol-5-yl)cyclopentanol et son chlorhydrate**

Le produit est obtenu selon le procédé de l'exemple 38 des stades A à C, en utilisant au stade A le produit de la préparation 15 et au stade B le chlorure de l'acide (2S)-2,3-dihydrobenzo[1,4]dioxin-2-yl carboxylique.
**Point de fusion (chlorhydrate) : 109-119°C (M.K.)**

**EXEMPLE 40 :**

**1-(1-{[[2-(5-Fluoro-1*H*-indol-3-yl)éthyl](méthyl)amino]méthyl}-1,2-dihydrocyclobuta[*b*]naphthalèn-1-yl)cyclopentanol**

***Stade A** : 1-{[(Méthyl)amino]méthyl}-1,2-dihydrocyclobuta[*b*]naphthalèn-1-yl) cyclopentanol*

Le produit est obtenu selon le procédé de l'exemple 22 en utilisant comme substrat le 1-[1-(arninométhyl)-1,2-dihydrocyclobuta[*b*]naphthalèn-1-yl]cyclopentanol qui est obtenu à partir de la préparation 17

***Stade B** : 2-(5-Fluoro-1H-indol-3-yl)***N**-*[[1-(1-hydroxycyclopentyl) -1,2-dihydrocyclobuta[*b*]naphthalèn-1-yl]méthyl]***N**-*méthylacétamide*

0,652 g d'acide (5-fluoro-indol-3-yl)acétique dissous dans 30 ml de dichlorométhane sont traités par 0,61 g de carbonyldiimidazole, à température ambiante pendant 30 minutes. Une solution de 0,95 g du produit obtenu au stade A dissous dans 5 ml de dichlorométhane sont coulés sur la solution. En fin de réaction, le milieu réactionnel est repris par de l'eau, décanté, séché, évaporé, pour conduire au produit attendu.

***Stade C** : 1-(1-{[[2-(5-fluoro-1H-indol-3 -yl)éthyl](méthyl)amino]méthyl}-1,2-dihydrocyclobuta[*b*]naphthalèn-1-yl)cyclopentanol*

Le produit est obtenu selon le procédé de l'exemple 39 en utilisant comme substrat le produit obtenu au stade B précédent.

***ETUDE PHARMACOLOGIOUE DES COMPOSES DE L'INVENTION***

***A. Etudes in vitro***

**EXEMPLE 41 :**

**Détermination de l'affinité pour les sites de recapture de la sérotonine**

L'affinité a été déterminée par des expériences de compétition avec la [$^3$H]-paroxetine (NEN, Les Ulis, France). Les membranes sont préparées à partir de cortex frontal de rat et incubées en triple avec 1,0 nM de [$^3$H]-paroxetine et le ligand froid dans un volume final de 0,4 ml, pendant 2 heures à 25° C. Le tampon d'incubation contient 50 nM de TRIS-HCl (pH 7,4), 120 mM de NaCl et 5 mM de KCI. La fixation non-spécifique est déterminée avec 10 µM de citalopram. A la fin de l'incubation, le milieu d'incubation est filtré et lavé trois fois avec 5 ml de tampon refroidi. La radioactivité retenue sur les filtres est déterminée par comptage du liquide de scintillation. Les isothermes de binding sont analysées par régression non-linéaire pour déterminer les valeurs d'$IC_{50}$. Elles sont converties en constante de dissociation ($K_i$) par l'intermédiaire de l'équation de Cheng-Prusoff :

$$K_i = IC_{50} / (1 + L / K_d)$$

dans laquelle L est la concentration de [$^3$H]-paroxetine et $K_d$ est la constante de dissociation de [$^3$H]-paroxetine pour le site de recapture de la sérotonine (0,13 nM). Les résultats sont exprimés en $pK_1$ (- log $K_i$).

Les composés de la présente invention montrent une très bonne affinité pour les sites de recapture de la sérotonine, leur $pK_i$ est ≥ 7.

**EXEMPLE 42 :**

**Détermination de l'affinité pour les sites de recapture de la noradrénaline**

L'affinité a été déterminée par des expériences de compétition avec la [$^3$H]-nisoxétine (Amersham, les Ulis, France). Les membranes sont préparées à partir de cortex frontal de rat et incubées en triple avec 2 nM de [$^3$H]-nisoxétine et le ligand froid dans un volume final de 0,5 ml, pendant 4 heures à 4° C. Le tampon d'incubation contient 50 mM de TRIS-HC1 (pH 7,4), 300 mM de NaCl et 5 mM de KCl. La fixation non-spécifique est déterminée avec 10 µM de desipramine. A la fin de l'incubation, le milieu d'incubation est filtré et lavé trois fois avec 5 ml de tampon de filtration refroidi (50 mM de TRIS-HCl, pH 7,4, 300mM de NaCl et 5 mM de KCl). La radioactivité retenue sur les filtres est déterminée par comptage du liquide de scintillation. Les isothermes de binding sont analysées par régression non-linéaire pour déterminer les valeurs d'$IC_{50}$. Elles sont converties en constante de dissociation ($K_i$) par l'intermédiaire de l'équation de Cheng-Prusoff:

$$K_i = IC_{50} / (1 + L/K_d)$$

dans laquelle L est la concentration de [$^3$H]-nisoxetine et $K_d$ est la constante de dissociation de [$^3$H]-nisoxetine pour le site de recapture de la noradrénaline (1,23 nM). Les résultats sont exprimés en pKi (-log Ki).

Le pKi des composés de l'invention est ≥ 6.

***B. Etudes in vivo***

**EXEMPLE 43 :**

**Expérience de la microdialyse chez le rat**

Les rats sont anesthésiés au pentobarbital (60 mg/kg i.p.). Ils sont placés dans un appareil stéréotaxique de Kopf et le guide de la canule est implanté dans le cortex frontal cingulé suivant les coordonnées décrites comme suit dans l'atlas de Paxinos et Watson (1982) : AP = + 2,2 ; L = ± 0,6 ; DV = - 0,2. Les rats sont mis en cage séparément et ne sont utilisés en dialyse que 5 jours plus tard. Le jour de la dialyse la sonde est descendue lentement et maintenue dans sa position. La sonde est perfusée à un débit de 1 µl/mn avec une solution de 147,2 mM de NaCl, 4 mM de KCl et 2,3 mM de $CaCl_2$ amené à pH 7,3 avec un tampon phosphate (0,1 M). Deux heures après l'implantation, les échantillons sont collectés toutes les 20 minutes pendant 4 heures. Trois échantillons de base sont collectés avant l'administration des produits à tester. Les rats sont laissés dans leur cage individuelle pendant toute l'expérience. A la fin de l'expérience, les rats sont décapités et le cerveau prélevé est congelé dans l'isopentane. Des sections d'une épaisseur de 100 μm sont coupées et colorées avec du cresyl violet, ce qui permet la vérification de l'emplacement des sondes. La quantification simultanée de dopamine, norépinéphrine et sérotonine est effectuée de la façon suivante : 20 μl d'échantillon de dialyse sont dilués avec 20 μl de phase mobile ($NaH_2PO_4$ : 75 mM, EDTA : 20 μM, sodium 1-decanesulfonate : 1 mM, méthanol : 17,5 %, triethylamine : 0,01 %, pH : 5,70) et 33 μl d'échantillons sont analysés par HPLC avec une colonne en phase inverse thermostatée à 45° C et quantifiés par l'intermédiaire d'un détecteur coulométrique. Le potentiel de la première électrode du détecteur est fixée à - 90 mV (réduction) et la seconde à + 280 mV (oxydation). La phase mobile est injectée avec une pompe à un débit de 2 ml/mn. Les limites de sensibilité pour la dopamine, la norépinéphrine et la sérotonine sont de 0,55 finole par échantillon. Tous les produits de l'invention sont injectés par voie sous-cutanée dans un volume de 1,0 ml/kg. Les produits sont dissous dans de l'eau distillée additionnée de quelques gouttes d'acide lactique si nécessaire.

***Résultats :***

A titre d'exemple et pour illustrer l'activité des produits de l'invention, le composé de l'exemple 5, administré à la dose de 10 mg/kg, par voie sous-cutanée, augmente le niveau de sérotonine de : 250 ± 15 %, celui de la noradrénaline de 500 ± 13 % et celui de la dopamine 400 ± *50 % (%* maximal de l'effet par rapport au niveau basal défini comme le 0 %).

**EXEMPLE 44 :**

**Test d'enfouissement des billes chez la souris**

Ce test permet d'évaluer la capacité d'agents pharmacologiques à inhiber le comportement spontané d'enfouissement de billes chez la souris, cette inhibition étant prédictive d'action antidépressive et/ou anti-impulsive. Des souris mâles de souche NMRI (Iffa-Credo, l'Arbresle, France), pesant 20-25 g le jour de l'expérience, sont placées individuellement dans des boîtes en Macrolon® (30 x 18 x 19 cm) contenant 5 cm de sciure et recouvertes par une plaque en plexiglass perforée. Vingt-quatre billes en verre "oeil-de-chat" sont réparties régulièrement sur la sciure à la périphérie de la boîte. Au terme de 30 minutes d'exploration libre, les animaux sont retirés de la boîte et le nombre de billes enfouies est comptabilisé.

***Résultats :***

A titre d'exemple, le tableau montre l'effet d'un produit de l'invention, en comparaison avec celui de la fluoxétine, un antidépresseur de référence.

| ***Exemple*** | ***Enfouissement des billes chez la souris $DI_{50}$*** |
|---|---|
| Fluoxétine | 8,03 |
| 5 | 0,6 |
| $DI_{50}$ = dose inhibitrice$_{50}$ | |

Les doses sont exprimées en mg/kg s.c.

**EXEMPLE 45 :**

**Composition pharmaceutique : comprimés**

| *Formule de préparation pour 1000 comprimés dosés à 5 mg* | |
|---|---|
| Composé de l'exemple 5 | g |
| Hydroxypropylméthylcellulose | 5 g |
| Amidon de blé | 10 g |

(suite)

| | |
|---|---|
| Lactose | 100 g |
| Stéarate de Magnésium | 2 g |

## Revendications

**1.** Composés de formule (I) :

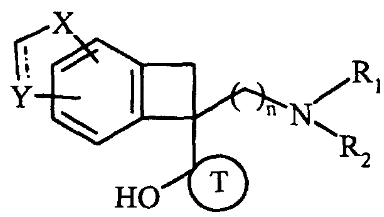

**(I)**

dans laquelle :

----- représente une liaison simple ou une liaison double,

n représente 1,

$R_1$, $R_2$, identiques ou différents, indépendamment l'un de l'autre représentent un groupement choisi parmi atome d'hydrogène, groupement alkyle ($C_1$-$C_6$) linéaire ou ramifié et 2,3-dihydro-1,4-benzodioxin-2-ylméthyle,

X représente un groupement choisi parmi -CH=CH-, atome d'oxygène, groupement $S(O)_m$ dans lequel m est un entier compris entre 0 et 2 inclus, et $NR_3$ dans lequel $R_3$ représente un groupement choisi parmi atome d'hydrogène, groupement alkyle ($C_1$-$C_6$) linéaire ou ramifié, aryle, arylalkyle ($C_1$-$C_6$) linéaire ou ramifié, cycloalkyle, cycloalkylalkyle ($C_1$-$C_6$) linéaire ou ramifié, alkényle ($C_2$-$C_6$) linéaire ou ramifié, et alkynyle ($C_2$-$C_6$) linéaire ou ramifié,

Y représente un groupement CH ou $CH_2$ selon que -------- représente une liaison simple ou une liaison double, ou peut prendre la définition supplémentaire atome d'oxygène quand X représente un atome d'oxygène,

T représente un groupement cyclopentyle ou cyclohexyle,

leurs isomères optiques ainsi que leurs sels d'addition à un acide ou à une base pharmaceutiquement acceptable,

étant entendu que :

- par groupement aryle, on comprend un groupement choisi parmi phényle, biphényle, naphtyle, dihydronaphtyle, tétrahydronaphtyle, indanyle, indényle, et benzocyclobutyle, chacun de ces groupements pouvant être éventuellement substitués par un ou plusieurs groupements, identiques ou différents, choisis parmi atomes d'halogène, groupements alkyle ($C_1$-$C_6$) linéaire ou ramifié, hydroxy, alkoxy ($C_1$-$C_6$) linéaire ou ramifié, nitro, cyano, trihalogénoalkyle ($C_1$-$C_6$) linéaire ou ramifié, amino, monoalkylamino et dialkylamino ($C_1$-$C_6$) linéaire ou ramifié,
- par groupement cycloalkyle, on comprend un système mono ou polycyclique, de 3 à 12 chaînons, contenant éventuellement une ou plusieurs insaturations, celles-ci ne conférant pas de caractère aromatique audit système cyclique.

**2.** Composés de formule (I) selon la revendication 1 **caractérisés en ce qu'**ils représentent des composés de formule **(I/A) :**

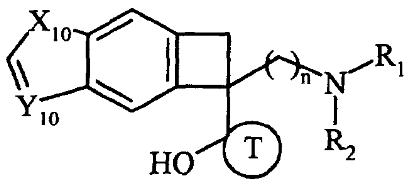

(I/A)

dans laquelle n, $R_1$, $R_2$ et T sont tels que définis dans la formule (I), $X_{10}$ représente un atome d'oxygène ou un atome de soufre, et $Y_{10}$ représente un groupement CH, leurs isomères ainsi que leurs sels d'addition à un acide ou à une base pharmaceutiquement acceptable.

**3.** Composés de formule (I) selon la revendication 1 **caractérisés en ce qu'**ils représentent des composés de formule **(I/B) :**

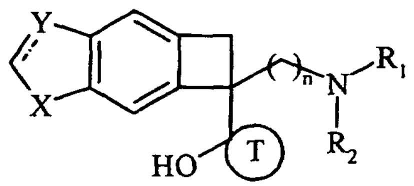

(I/B)

dans laquelle X, Y, n, $R_1$, $R_2$, $R_3$ et T sont tels que définis dans la formule (I), leurs isomères ainsi que leurs sels d'addition à un acide ou à une base pharmaceutiquement acceptable.

**4.** Composés de formule (I/B) selon la revendication 3 **caractérisé en ce que** X représente un groupement $NR_3$ dans lequel $R_3$ représente un atome d'hydrogène ou un groupement alkyle ($C_1$-$C_6$) linéaire ou ramifié, leurs isomères ainsi que leurs sels d'addition à un acide ou à une base pharmaceutiquement acceptable.

**5.** Composés de formule (I) selon la revendication 1 **caractérisés en ce qu'**ils représentent des composés de formule **(I/C) :**

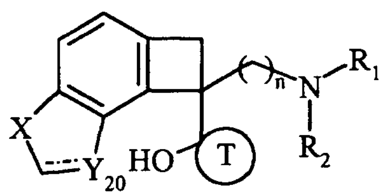

(I/C)

dans laquelle n, $R_1$, $R_2$, X et T sont tels que définis dans la formule (I) et $Y_{20}$ représente un groupement CH ou $CH_2$, leurs isomères ainsi que leurs sels d'addition à un acide ou à une base pharmaceutiquement acceptable.

**6.** Composés de formule (I) selon la revendication 1 **caractérisés en ce qu'**ils représentent des composés de formule **(I/D) :**

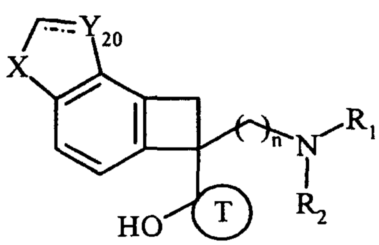

(I/D)

dans laquelle n, $R_1$, $R_2$, X et T sont tels que définis dans la formule (I) et $Y_{20}$ représente un groupement CH ou

$CH_2$, leurs isomères ainsi que leurs sels d'addition à un acide ou à une base pharmaceutiquement acceptable.

**7.** Composés de formule (I) selon la revendication 1 **caractérisés en ce qu'**ils représentent des composés de formule **(I/E) :**

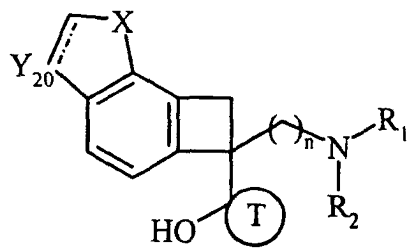

**(I/E)**

dans laquelle n, $R_1$, $R_2$, X et T sont tels que définis dans la formule (I) et $Y_{20}$ représente un groupement CH ou $CH_2$, leurs isomères ainsi que leurs sels d'addition à un acide ou à une base pharmaceutiquement acceptable.

**8.** Composés de formule (I) selon la revendication 1 **caractérisés en ce qu'**ils représentent des composés de formule **(I/F) :**

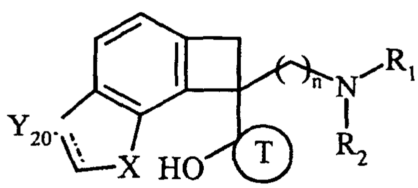

**(I/F)**

dans laquelle n, $R_1$, $R_2$, X et Y sont tels que définis dans la formule (I) et $Y_{20}$ représente un groupement CH ou $CH_2$, leurs isomères ainsi que leurs sels d'addition à un acide ou à une base pharmaceutiquement acceptable.

**9.** Composés de formule (I) selon la revendication 1 qui sont le :

- 1-{6-[(diméthylamino)méthyl]-1-méthyl-2,3,5,6-tétrahydro-1*H*-cyclobuta[*f*]indol-6-yl} cyclohexanol,
- 1-{6-[(diméthylamino)méthyl]-2,3,5,6-tétrahydro-1*H*-cyclobuta[*f*]indol-6-yl} cyclohexanol,
- le 1-{5-[(diméthylamino)méthyl]-5,6-dihydrocyclobuta[*f*][1]benzofuran-5-yl}cyclohexanol,
- 1-{5-[(diméthylamino)méthyl]-5,6-dihydrocyclobuta[*f*][*1*]benzothien-5-yl} cyclohexanol,
- 1-{6-[(diméthylamino)méthyl]-1-méthyl-5,6-dihydro-1*H*-cyclobuta[*f*]indol-6-yl} cyclohexanol,
- 1-{7-[(diméthylamino)méthyl]-2,3,6,7-tétrahydro-1*H*-cyclobuta[*e*]indol-7-yl} cyclohexanol,
- 1-{5-[(méthylamino)méthyl]-5,6-dihydrocyclobuta[*f*][1]benzothièn-5-yl} cyclopentanol,
- 1-{5-[(diméthylamino)méthyl]-5,6-dihydrocyclobuta[*f*][1]benzothièn-5-yl} cyclopentanol,
- (+)1-{5-[(diméthylamino)méthyl]-5,6-dihydrocyclobuta[*f*][1]benzothièn-5-yl} cyclopentanol,
- (-)1-{5-[(diméthylamino)méthyl]-5,6-dihydrocyclobuta[*f*][1]benzothièn-5-yl} cyclopentanol,
- 1-{6-[(diméthylamino)méthyl]-5,6-dihydrocyclobuta[*f*][1]benzothièn-6-yl} cyclopentanol,
- 1-{5-[(diméthylamino)méthyl]-1-méthyl-5,6-dihydro-1*H*-cyclobuta[*f*]indol-5-yl} cyclopentanol,
- 1-{7-[(diméthylamino)méthyl]-6,7-dihydrocyclobuta[*g*][1]benzofuran-7-yl} cyclopentanol,
- 1-{1-[(diméthylamino)méthyl]-1,2-dihydrocyclobuta[*b*]naphthalèn-1-yl}cyclopentanol,
- 1-{7-[(diméthylamino)méthyl]-6,7-dihydro-3*H*-cyclobuta[*e*]indol-7-yl}cyclopentanol,
- 1-{1-[(dimethylamino)methyl]-1,2-dihydrocyclobuta[*a*]naphthalèn-1-yl}cyclopentanol. leurs isomères ainsi que leurs sels d'addition à un acide ou à une base pharmaceutiquement acceptable.

**10.** Procédé de préparation des composés de formule (I), **caractérisé en ce qu'**on utilise comme produit de départ :

***a)*** ***<u>soit</u>*** un composé de formule **(II) :**

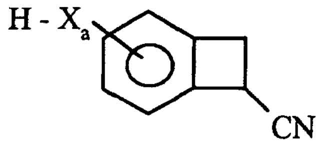

**(II)**

dans laquelle $X_a$ représente un atome de soufre, d'oxygène, ou un groupement NH, composé de formule (II) que l'on fait réagir avec un composé de formule **(III) :**

$$Z - CH(OA)_2 \qquad \textbf{(III)}$$

dans laquelle A représente un groupement alkyle ($C_1$-$C_4$) linéaire ou ramifié et Z représente un groupement formyle (dans le cas où $X_a$ représente un groupement NH), ou un groupement $-CH_2Hal$ dans lequel Hal représente un atome de chlore, de brome ou d'iode,
pour conduire aux composés de formule **(IV) :**

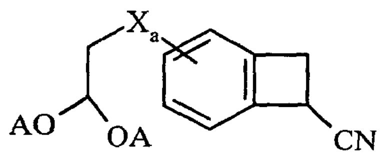

**(IV)**

dans laquelle $X_a$ et A sont tels que définis précédemment,
composés de formule (IV) qui sont soumis :

- <u>soit dans le cas où $X_a$ prend la définition $X_1$</u> représentant un atome d'oxygène ou un atome de soufre, à des conditions de cyclisation par action d'un acide tel que l'acide polyphosphorique ou un acide de Lewis, pour conduire aux composés de formule **(V/a) :**

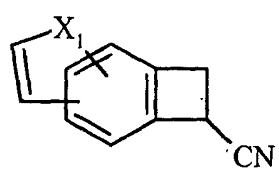

**(V/a)**

dans laquelle $X_1$ représente un atome d'oxygène ou de soufre,
composés de formule (V/a) qui sont traités :
en présence d'une base forte, par une cétone cyclique de formule **(VI) :**

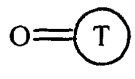

**(VI)**

dans laquelle T a les mêmes significations que dans la formule (I), pour conduire aux composés de formule **(VII/a),**

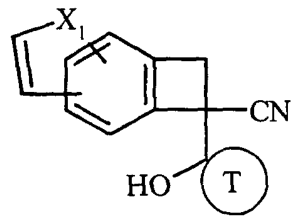

**(VII/a)**

dans laquelle $X_1$ et T sont tels que définis précédemment,
composés de formule (VII/a) qui sont soumis à l'action d'un agent réducteur selon des conditions classiques de la synthèse organique, pour conduire aux composés de formule **(I/a),** cas particulier des composés de formule **(I)** :

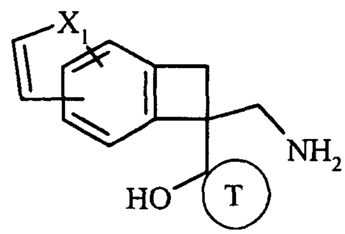

**(I/a)**

dans laquelle $X_1$ et T sont tels que définis précédemment,
composés de formule (I/a) pour lesquels :

➪ ***soit*** on substitue la fonction amine primaire selon des méthodes classiques de la synthèse organique, telle que l'amination réductrice ou la substitution nucléophile de dérivé de formule **(VIIIa) :**

$$R'_1 - Z_1 \qquad \textbf{(VIIIa)}$$

dans laquelle $R'_1$ a les mêmes définitions que $R_1$ dans la formule (I) exceptées la valeur atome d'hydrogène, et $Z_1$ représente un groupe partant usuel de la chimie organique tel que atome d'halogène, groupement mésylate ou tosylate,
pour conduire aux composés de formule **($I/b_1$),** cas particulier des composés de formule **(I) :**

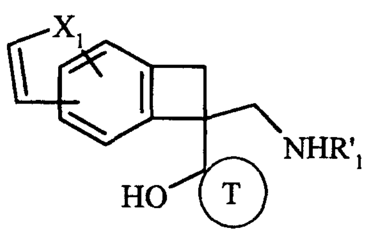

**($I/b_1$)**

dans laquelle $X_1$, T et $R'_1$ sont tels que définis précédemment,

➪ ***soit*** selon une variante avantageuse du procédé, on traite par un dérivé de formule **(VIIIb) :**

$$R''_1 - COZ_2 \qquad \textbf{(VIIIb)}$$

dans laquelle $R''_1$ représente un groupement hétérocycloalkylalkyle ($C_1$-$C_5$) linéaire ou ramifié, et $Z_2$ représente un atome de chlore ou un groupement imidazolyle, pour conduire aux composés de formule **($I/b_2$) :**

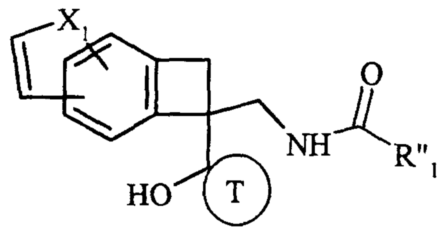

(I/b$_2$)

dans laquelle $X_1$, T et $R''_1$ sont tels que définis précédemment, composés de formule (I/$b_2$) qui sont réduits par un agent réducteur classiquement utilisé en chimie organique, pour conduire aux composés de formule **(I/$b_3$) :**

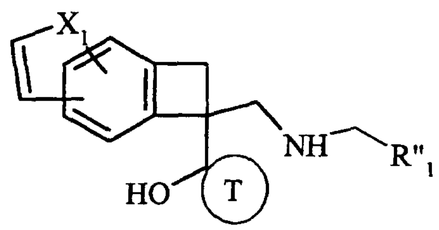

(I/b$_3$)

dans laquelle $X_1$, $R''_1$ et T sont tels que définis précédemment, l'ensemble des composés de formule (I/$b_1$) et (I/$b_3$) forment les composés de formule (I/b), composés de formule (I/b) qui sont traités selon les mêmes conditions que décrites précédemment par un composé de formule **(VIIIc) :**

$$R'_2\text{-}Z_1 \qquad \textbf{(VIIIc)}$$

dans laquelle $Z_1$ est tel que défini précédemment et $R'_2$ prend les mêmes définitions que $R_2$ dans la formule (I) à l'exception de la valeur atome d'hydrogène, pour conduire aux composés de formule **(I/c),** cas particulier des composés de formule (I) :

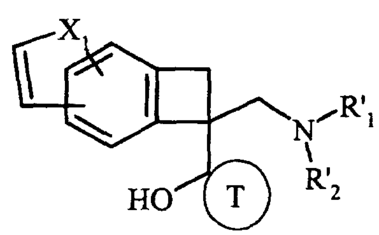

(I/c)

dans laquelle $X_1$, T, $R'_1$ et $R'_2$ sont tels que définis précédemment, l'ensemble des composés de formule (I/a), (I/b) et (I/c), dans le cas particulier où $X_1$ représente un atome de soufre, forment les composés de formule **(I/e) :**

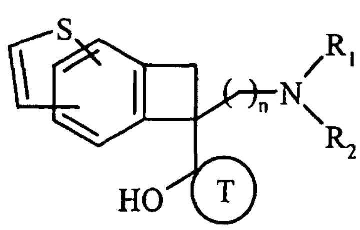

(I/e)

dans laquelle n, $R_1$, $R_2$ et T sont tels que définis dans la formule (I),

composés de formule (I/e) qui sont soumis à l'action d'un agent d'oxydation selon des conditions classiques de la synthèse organique, pour conduire aux composés de formule **(I/f),** cas particulier des composés de formule (I) :

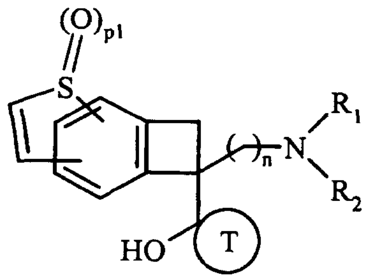

**(I/f)**

dans laquelle $p_1$ est un entier choisi parmi 1 et 2, et n, $R_1$, $R_2$ et T sont tels que définis précédemment, l'ensemble des composés de formule (I/a), (I/b), (I/c) (dans lesquels $X_1$ représente un atome d'oxygène ou de soufre) et (I/f) forment les composés de formule **(I/g) :**

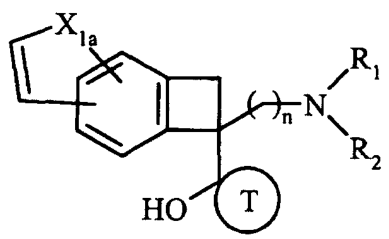

**(I/g)**

dans laquelle $X_{1a}$ représente un atome d'oxygène ou un groupement de formule $S(O)_p$ avec p tel que défini dans la formule (I), et n, $R_1$, $R_2$ et T sont tels que définis dans la formule (I), composés de formule (I/g) qui sont soumis à l'action d'un agent réducteur selon des conditions classiques de la synthèse organique, pour conduire aux composés de formule **(I/h),** cas particulier des composés de formule (I) :

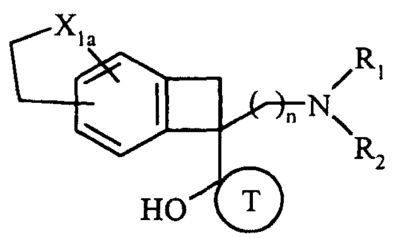

**(I/h)**

dans laquelle $X_{1a}$, n, $R_1$, $R_2$ et T sont tels que définis précédemment,

- soit, dans le cas où $X_a$ prend la définition $X'_2$ représentant un groupement NH, à l'action d'un chlorure d'acide sulfonique de formule **(XVI) :**

$$E - SO_2Cl \qquad \textbf{(XVI)}$$

dans laquelle E représente un groupement alkyle ($C_1$-$C_4$) linéaire ou ramifié, phényle ou p-toluyle, pour conduire aux composés de formule **(XVII) :**

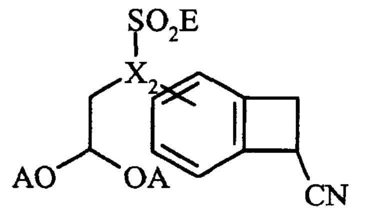

**(XVII)**

dans laquelle $X_2$ représente un atome d'azote, E et A sont tels que définis précédemment, composés de formule (XVII) qui sont cyclisés par action d'un acide pour conduire aux composés de formule **(V/b) :**

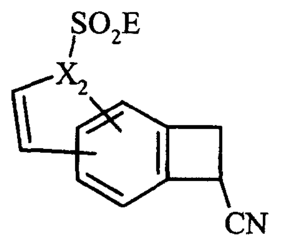

**(V/b)**

dans laquelle $X_2$ et E sont tels que définis précédemment,
composés de formule (V/b) dont on déprotège l'amine cyclique par action d'un agent basique, puis que l'on soumet à l'action d'un agent réducteur selon des conditions classiques de la synthèse organique, pour conduire aux composés de formule **(V/c) :**

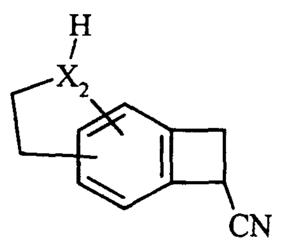

**(V/c)**

dans laquelle $X_2$ est tel que défini précédemment,
composés de formule (V/c) qui sont soumis à l'action d'un composé de formule (VI) tel que décrit précédemment pour conduire aux composés de formule **(VIIIb) :**

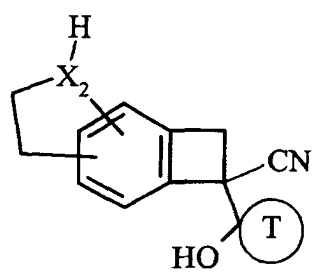

**(VII/b)**

dans laquelle $X_2$ est tel que défini précédemment et T est tel que défini dans la formule (I), composés de formule (VII/b) qui sont :

- ***soit*** traités selon les mêmes conditions que celles décrites pour les composés de formule (VII/a), pour conduire aux composés de formule **(I/i),** cas particulier des composés de formule (I) :

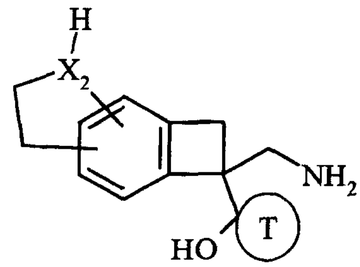

**(I/i)**

dans laquelle $X_2$ et T sont tels que définis précédemment,
composés de formule (I/i) qui peuvent être traités successivement par un composé de formule (VIIIa) ou (VIIIb), puis (VIIIc), tels que définis précédemment, pour conduire respectivement aux composés de formule **(I/j)** et **(I/k),** cas particuliers des composés de formule (I) :

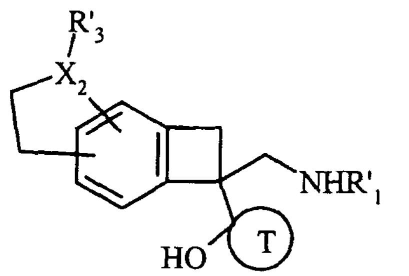

,

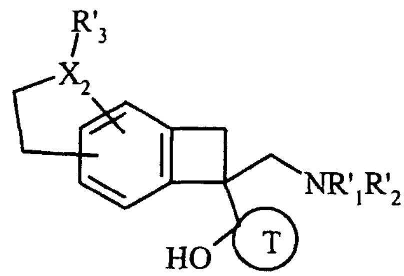

**(I/j)** **(I/k)**

dans lesquelles $X_2$, T, $R'_1$ et $R'_2$ sont tels que définis précédemment, et $R'_3$ a les mêmes définitions et valeurs que $R'_1$,
l'ensemble des composés de formules (I/i), (I/j), (I/k) forment les composés de formule **(I/l) :**

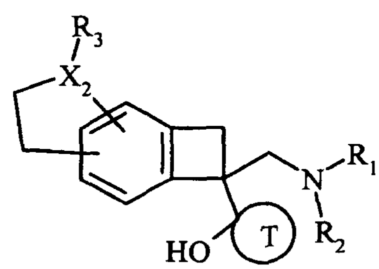

**(I/l)**

dans laquelle $X_2$, $R_1$, $R_2$, $R_3$ et T sont tels que définis dans la formule (I),
composés de formule (I/l) qui sont soumis à l'action d'un agent oxydant tel que le dioxyde de manganèse, pour conduire aux composés de formule **(I/m),** cas particulier des composés de formule **(I)** :

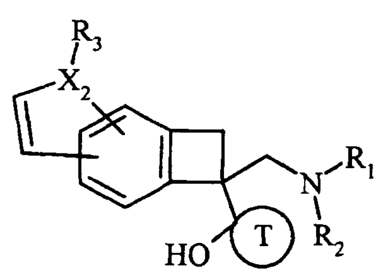

**(I/m)**

dans laquelle $X_2$, $R_1$, $R_2$, $R_3$ et T sont tels que définis précédemment,

- ***soit*** traités par un composé de formule (XVI) telle que définie précédemment, pour conduire aux composés de formule **(XVIII) :**

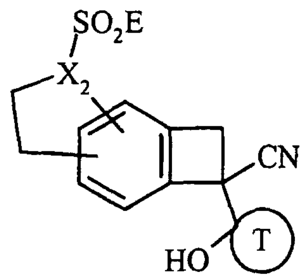

**(XVIII)**

dans laquelle T, $X_2$ et E sont tels que définis précédemment,
composés de formule (XVIII) qui sont réduits selon des conditions classiques de la synthèse organique, pour conduire aux composés de formule **(XIX) :**

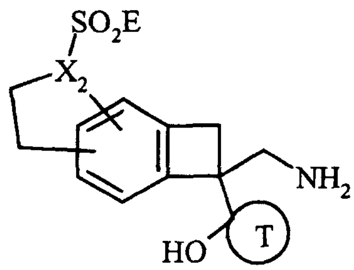

**(XIX)**

dans laquelle T, $X_2$ et E sont tels que définis précédemment,
composés de formule (XIX) dont la fonction amine primaire peut être substituée par action d'un composé de formule ($VIII_a$) tel que défini précédemment, pour conduire aux composés de formule **(XX) :**

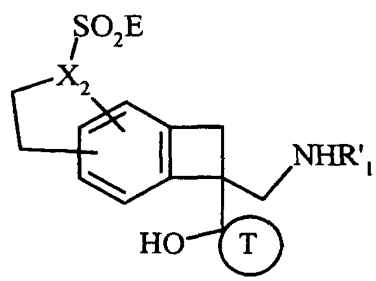

**(XX)**

dans laquelle T, $X_2$, E et $R'_1$ ont les mêmes significations que celles décrites précédemment,
composés de formule (XX) qui peuvent être transformés en amine tertiaire par action d'un composé de formule ($VIII_b$) telle que définie précédemment, pour conduire aux composés de formule **(XXI) :**

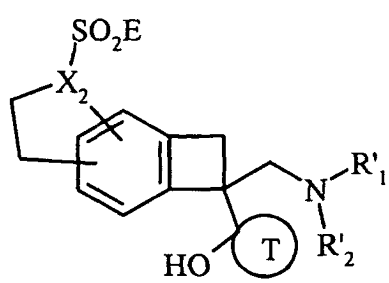

**(XXI)**

dans laquelle T, $X_2$, E, $R'_1$ et $R'_2$ sont tels que définis précédemment,
composés de formule (XX) et (XXI) qui sont ensuite déprotégés par traitement avec du sodium dans l'ammoniaque liquide, pour conduire respectivement aux composés de formules **(I/n)** et **(I/o),** cas particuliers des composés de formule (I) :

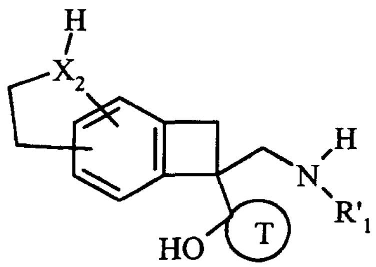

,

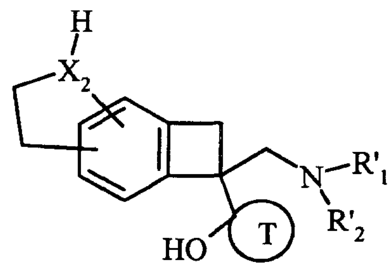

**(I/n)** **(I/o)**

dans laquelle T, $X_2$, $R'_1$ et $R'_2$ sont tels que définis précédemment,

**b)** ***soit*** un composé de formule **(II/1) :**

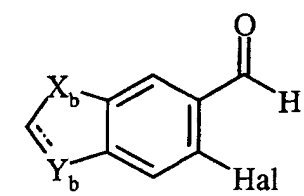

**(II/1)**

dans laquelle Hal représente un atome d'halogène, et $X_b$ représente un atome d'oxygène quand $Y_b$ représente un atome d'oxygène et ----- représente une liaison simple, ou $X_b$ représente un groupement -CH=CH- quand $Y_b$ représente un groupement CH et ----- représente une liaison double,
composés de formule (II/1) qui est mis à réagir avec $(EtO)_2POCH_2CN$ pour conduire aux composés de formule (II/2) :

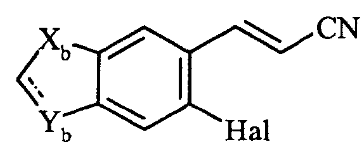

**(II/2)**

dans laquelle Hal, $X_b$ et $Y_b$ sont tels que définis précédemment,
composés de formule (II/2) qui est d'abord soumis à l'action d'un réducteur classique de la chimie organique puis qui est mis à réagir avecNaNH$_2$, pour conduire aux composés de formule (II/3) :

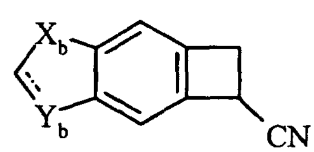

**(II/3)**

dans laquelle Xb et Yb sont tels que définis précédemment,
composés de formule (II/3) qui peuvent être traités dans les mêmes conditions que les composés de formule (V/a) par un composé de formule (VI), puis (VIIIa) ou (VIIIb), puis (VIIIc), pour conduire successivement aux

composés de formules ($I/3_a$), ($I/3_b$) et ($I/3_c$) :

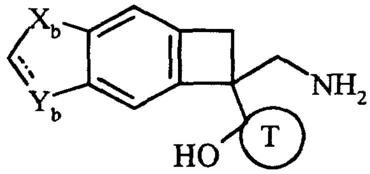

,

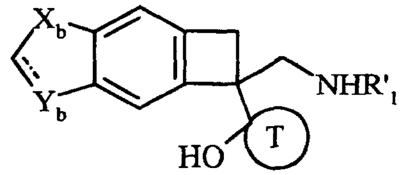

($I/3_a$)

($I/3_b$)

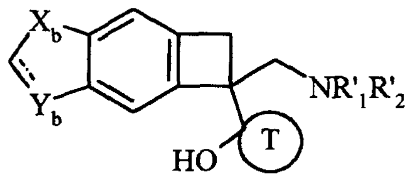

($I/3_c$)

dans lesquelles $X_b$, $Y_b$, T, $R'_1$ et $R'_2$ sont tels que définis précédemment, les composés (I/a) à (I/o) et ($I/3_a$) à ($I/3_c$) forment l'ensemble des composés de l'invention, que l'on purifie le cas échéant, selon une technique classique de purification, qui peuvent, si on le désire, être séparés en leurs différents isomères selon une technique classique de séparation, et que l'on transforme, le cas échéant, en leurs sels d'addition à un acide ou à une base pharmaceutiquement acceptable.

**11.** Compositions pharmaceutiques contenant comme principe actif au moins un composé de formule (I) selon l'une quelconque des revendications 1 à 9, seul ou en combinaison avec un ou plusieurs excipients ou véhicules inertes, non toxiques, pharmaceutiquement acceptables.

**12.** Compositions pharmaceutiques selon la revendication 11 contenant au moins un principe actif selon l'une quelconque des revendications 1 à 9, utiles en tant que médicament, dans le traitement de la dépression, des attaques de panique, des troubles obsessionnels compulsifs, des phobies, des troubles impulsifs, de l'abus de drogue, de l'anxiété, de l'obésité et de la boulimie.

**Patentansprüche**

**1.** Verbindungen der Formel (I):

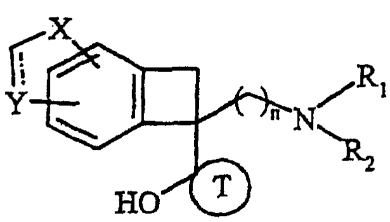

(I)

in der:

------ eine Einfachbindung oder eine Doppelbindung darstellt,
n 1 bedeutet,
$R_1$ und $R_2$, die gleichartig oder verschieden sind, unabhängig voneinander eine Gruppe ausgewählt aus dem

Wasserstoffatom, geradkettigen oder verzweigten ($C_1$-$C_6$)-Alkylgruppen und der 2,3-Dihydro-1,4-benzodioxin-2-yl-methyl-gruppe bedeuten,
X eine Gruppe bedeutet ausgewählt aus -CH=CH-, dem Sauerstoffatom, der Gruppe $S(O)_m$, worin m eine ganze Zahl mit einem Wert zwischen 0 und 2 einschließlich bedeutet, und $NR_3$, worin $R_3$ eine Gruppe ausgewählt aus dem Wasserstoffatom, geradkettigen oder verzweigten ($C_1$-$C_6$)-Alkylgruppen, Arylgruppen, geradkettigen oder verzweigten Aryl-($C_1$-$C_6$)-alkylgruppen, Cycloalkylgruppen; geradkettigen oder verzweigten Cycloalkyl-($C_1$-$C_6$)-alkylgruppen, geradkettigen oder verzweigten ($C_2$-$C_6$)-Alkenylgruppen und geradkettigen oder verzweigten ($C_2$-$C_6$)-Alkinylgruppen darstellt,
Y eine Gruppe CH oder $CH_2$ in Abhängigkeit davon, ob ------- eine Einfachbindung oder eine Doppelbindung darstellt, bedeutet, oder die zusätzliche Definition eines Sauerstoffatoms annehmen kann, wenn X ein Sauerstoffatom bedeutet,
T eine Cyclopentyl- oder Cyclohexyl-gruppe darstellt,
deren optische Isomere sowie deren Additionssalze mit einer pharmazeutisch annehmbaren Säure oder Base,

mit der Maßgabe, daß:

- man unter einer Arylgruppe eine Gruppe ausgewählt aus Phenyl, Biphenyl, Naphthyl, Dihydronaphthyl, Tetrahydronaphthyl, Indanyl, Indenyl und Benzocyclobutyl versteht, wobei jede dieser Gruppen gegebenenfalls durch eine oder mehrere, gleichartige oder verschiedenartige Gruppen ausgewählt aus Halogenatomen, geradkettigen oder verzweigten ($C_1$-$C_6$)-Alkylgruppen, Hydroxygruppen, geradkettigen oder verzweigten ($C_1$-$C_6$) -Alkoxygruppen, Nitrogruppen, Cyanogruppen, geradkettigen oder verzweigten ($C_1$-$C_6$)-Trihalogenalkylgruppen, Aminogruppen, geradkettigen oder verzweigten ($C_1$-$C_6$)-Monoalkylaminogruppen und ($C_1$-$C_6$)-Dialkylaminogruppen substituiert sein können,
- man unter einer Cycloalkylgruppe ein mono- oder polycyclisches System mit 3 bis 12 Kettengliedern versteht, welches gegebenenfalls eine oder mehrere Unsättigungen aufweist, welche jedoch dem cyclischen System keinen aromatischen Charakter verleihen.

2. Verbindungen der Formel (I) nach Anspruch 1, **dadurch gekennzeichnet, daß** sie die Verbindungen der Formel **(I/A)** darstellen:

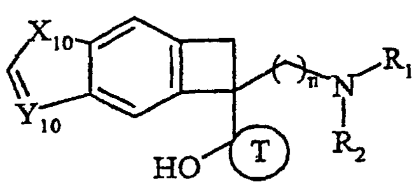

(I/A)

in der n, $R_1$, $R_2$ und T die bezüglich der Formel (I) angegebenen Bedeutungen besitzen, $X_{10}$ ein Sauerstoffatom oder ein Schwefelatom und $Y_{10}$ eine CH-Gruppe bedeuten, deren Isomere sowie deren Additionssalze mit einer pharmazeutisch annehmbaren Säure oder Base.

3. Verbindungen der Formel (I) nach Anspruch 1, **dadurch gekennzeichnet, daß** sie die Verbindungen der Formel **(I/B)** darstellen:

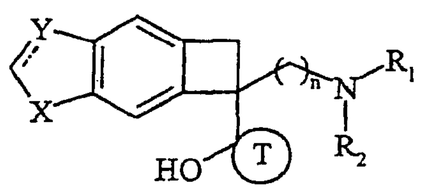

(I/B)

in der X, Y, n, $R_1$, $R_2$, $R_3$ und T die bezüglich der Formel (I) angegebenen Bedeutungen besitzen, deren Isomere sowie deren Additionssalze mit einer pharmazeutisch annehmbaren Säure oder Base.

4. Verbindungen der Formel (I/B) nach Anspruch 3, **dadurch gekennzeichnet, daß** X eine Gruppe $NR_3$ darstellt, in der $R_3$ ein Wasserstoffatom oder eine geradkettige oder verzweigte ($C_1$-$C_6$)-Alkylgruppe bedeutet, deren Isomere

sowie deren Additionssalze mit einer pharmazeutisch annehmbaren Säure oder Base.

5. Verbindungen der Formel (I) nach Anspruch 1, **dadurch gekennzeichnet, daß** sie die Verbindungen der Formel **(I/C)** darstellen:

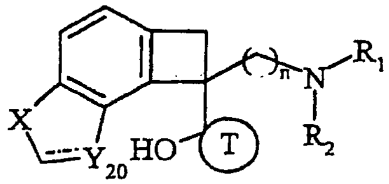

(I/C)

in der n, $R_1$, $R_2$, X und T die bezüglich der Formel (I) angegebenen Bedeutungen besitzen und $Y_{20}$ eine CH- oder $CH_2$-Gruppe bedeutet, deren Isomere sowie deren Additionssalze mit einer pharmazeutisch annehmbaren Säure oder Base.

6. Verbindungen der Formel (I) nach Anspruch 1, **dadurch gekennzeichnet, daß** sie die Verbindungen der Formel **(I/D)** darstellen:

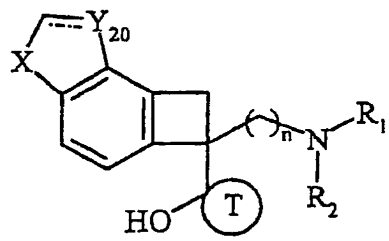

(I/D)

in der n, $R_1$, $R_2$, X und T die bezüglich der Formel (I) angegebenen Bedeutungen besitzen und $Y_{20}$ eine CH- oder $CH_2$-Gruppe bedeutet, deren Isomere sowie deren Additionssalze mit einer pharmazeutisch annehmbaren Säure oder Base.

7. Verbindungen der Formel (I) nach Anspruch 1, **dadurch gekennzeichnet, daß** sie die Verbindungen der Formel **(I/E)** darstellen:

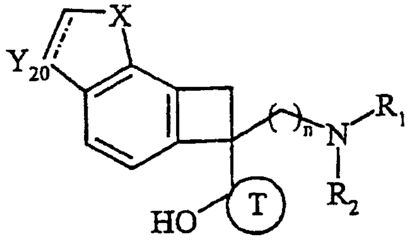

(I/E)

in der n, $R_1$, $R_2$, X und T die bezüglich der Formel (I) angegebenen Bedeutungen besitzen und $Y_{20}$ eine CH- oder $CH_2$-Gruppe bedeutet, deren Isomere sowie deren Additionssalze mit einer pharmazeutisch annehmbaren Säure oder Base.

8. Verbindungen der Formel (I) nach Anspruch 1, **dadurch gekennzeichnet, daß** sie die Verbindungen der Formel **(I/F)** darstellen:

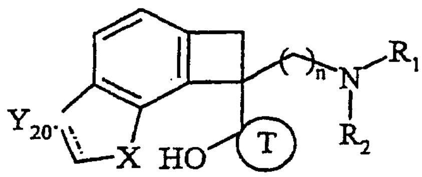

(I/F)

in der n, $R_1$, $R_2$, X und Y die bezüglich der Formel (I) angegebenen Bedeutungen besitzen und $Y_{20}$ eine CH- oder $CH_2$-Gruppe bedeutet, deren Isomere sowie deren Additionssalze mit einer pharmazeutisch annehmbaren Säure oder Base.

**9.** Verbindungen der Formel (I) nach Anspruch 1, nämlich:

- 1-{6-[(Dimethylamino)-methyl]-1-methyl-2,3,5,6-tetrahydro-1*H*-cyclobuta[*f*]indol-6-yl}-cyclohexanol,
- 1-{6-[(Dimethylamino)-methyl]-2,3,5,6-tetrahydro-1*H*-cyclobuta[*f*]indol-6-yl}-cyclohexanol,
- 1-{5-[(Dimethylamino)-methyl]-5,6-dihydrocyclobuta[*f*][1]benzofuran-5-yl}-cyclohexanol,
- 1-{5-[(Dimethylamino)-methyl]-5,6-dihydrocyclobuta[*f*][*1*]benzothien-5-yl}-cyclohexanol,
- 1-{6-[(Dimethylamino)-methyl]-1-methyl-5,6-dihydro-1*H*-cyclobuta[*f*]indol-6-yl}cyclohexanol,
- 1-{7-[(Dimethylamino)-methyl]-2,3,6,7-tetrahydro-1*H*-cyclobuta[*e*]indol-7-yl}-cyclohexanol,
- 1-{5-[(Methylamino]-methyl]-5,6-dihydrocyclobuta[*f*][1]benzothien-5-yl}-cyclopentanol,
- 1-{5-[(Dimethylamino)-methyl]-5,6-dihydrocyclobuta[*f*][1]benzothien-5-yl}-cyclopentanol,
- (+)-1-{5-[(Dimethylamino)-methyl]-5,6-dihydrocyclobuta[*f*][1]benzothien-5-yl}-cyclopentanol,
- (-)-1-{5-[(Dimethylamino)-methyl]-5,6-dihydrocyclobuta[*f*][1]benzothien-5-yl}-cyclopentanol,
- 1-{6-[(Dimethylamino)-methyl]-5,6-dihydrocyclobuta[*f*][1]benzothien-6-yl}-cyclopentanol,
- 1-{5-[(Dimethylamino)-methyl]-1-methyl-5,6-dihydro-1*H*-cyclobuta[*f*]indol-5-yl)-cyclopentanol,
- 1-{7-[(Dimethylamino)-methyl]-6,7-dihydrocyclobuta[*g*][1]benzofuran-7-yl}-cyclopentanol,
- 1-{1-[(Dimethylamino)-methyl]-1,2-dihydrocyclobuta[*b*]naphthalin-1-yl}-cyclopentanol,
- 1-{7-[(Dimethylamino)-methyl]-6,7-dihydro-3*H*-cyclobuta[*e*]indol-7-yl}-cyclopentanol,
- 1-{1-[(Dimethylamino)-methyl]-1,2-dihydrocyclobuta[*a*]naphthalin-1-yl}-cyclopentanol,

deren Isomere sowie deren Additionssalze mit einer pharmazeutisch annehmbaren Säure oder Base.

**10.** Verfahren zur Herstellung der Verbindungen der Formel (I), **dadurch gekennzeichnet, daß** man als Ausgangsprodukt:

***a)*** ***entweder*** eine Verbindung der Formel **(II)** verwendet:

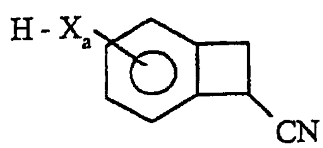

(II)

in der $X_a$ ein Schwefelatom, ein Sauerstoffatom oder eine Gruppe NH darstellt, welche Verbindung der Formel (II) man mit einer Verbindung der Formel **(III)** umsetzt:

$$Z - CH(OA)_2 \qquad \textbf{(III)}$$

in der A eine geradkettige oder verzweigte ($C_1$-$C_4$)-Alkylgruppe und Z eine Formylgruppe (wenn $X_a$ eine Gruppe NH bedeutet) oder eine Gruppe -$CH_2$Hal, worin Hal ein Chlor-, Brom- oder Iodatom darstellt, bedeuten, zur Bildung der Verbindungen der Formel **(IV):**

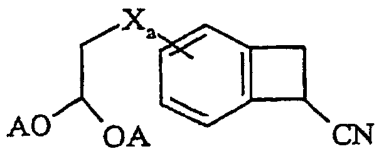

(IV)

in der $X_a$ und A die oben angegebenen Bedeutungen besitzen, welche Verbindungen der Formel (IV) man:

- entweder dann, $X_a$ die Bedeutung von $X_1$ aufweist, welche ein Sauerstoffatom oder ein Schwefelatom darstellt, den Bedingungen der Cyclisierung durch Einwirkung einer Säure, wie Polyphosphorsäure oder einer Lewis-Säure unterwirft zur Bildung der Verbindungen der Formel **(V/a):**

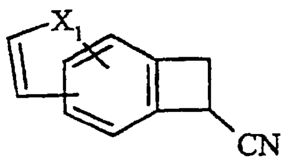

(V/a)

in der $X_1$ ein Sauerstoff- oder Schwefelatom bedeutet, welche Verbindungen der Formel (V/a) man:
in Gegenwart einer starken Base mit einem cyclischen Keton der Formel **(VI)** umsetzt:

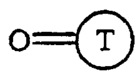

(VI)

in der T die bezüglich der Formel (I) angegebenen Bedeutungen besitzt, zur Bildung der Verbindungen der Formel **(VII/a):**

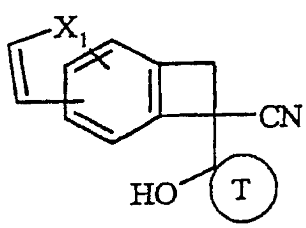

(VII/a)

in der $X_1$ und T die oben angegebenen Bedeutungen besitzen,
welche Verbindungen der Formel (VII/a) man der Einwirkung eines Reduktionsmittels unter klassischen Bedingungen der organischen Synthese unterwirft zur Bildung der Verbindungen der Formel **(I/a),** einem Sonderfall der Verbindungen der Formel (I):

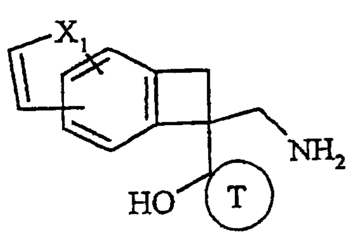

(I/a)

in der $X_1$ und T die oben angegebenen Bedeutungen besitzen,
welche Verbindungen der Formel (I/a) man:

⇨ ***entweder*** man an der primären Aminfunktion mit Hilfe klassischer Methoden der organischen

Synthese, wie durch reduzierende Aminierung oder durch nucleophile Substitution mit dem Derivat der Formel **(VIIIa):**

$$R'_1 - Z_1 \qquad \textbf{(VIIIa)}$$

in der $R'_1$ die gleichen Bedeutungen besitzt wie sie für $R_1$ bezüglich der Formel (I) angegeben sind mit Ausnahme der Bedeutung des Wasserstoffatoms, und $Z_1$ eine übliche austretende Gruppe der organischen Chemie darstellt, wie ein Halogenatom, eine Mesylat- oder Tosylatgruppe, substituiert, zur Bildung der Verbindungen der Formel **(I/b$_1$),** einem Sonderfall der Verbindungen der Formel (I):

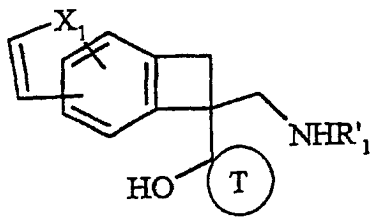

**(I/b$_1$)**

in der $X_1$, T und $R'_1$ die oben angegebenen Bedeutungen besitzen,

⇨ ***oder*** gemäß einer vorteilhaften Ausführungsform des Verfahrens mit einem Derivat der Formel **(VIIIb)** behandelt:

$$R''_1 - COZ_2 \qquad \textbf{(VIIIb)}$$

in der $R''_1$ eine geradkettige oder verzweigte Heterocycloalkyl-($C_1$-$C_5$)-alkylgruppe und $Z_2$ ein Chloratom oder eine Imidazolylgruppe bedeuten, zur Bildung der Verbindungen der Formel **(I/b$_2$):**

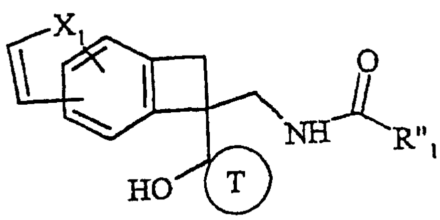

**(I/b$_2$)**

in der $X_1$, T und $R''_1$ die oben angegebenen Bedeutungen besitzen,

welche Verbindungen der Formel (I/b$_2$) man mit einem üblicherweise in der organischen Chemie verwendeten Reduktionsmittel reduziert zur Bildung der Verbindungen der Formel **(I/b$_3$):**

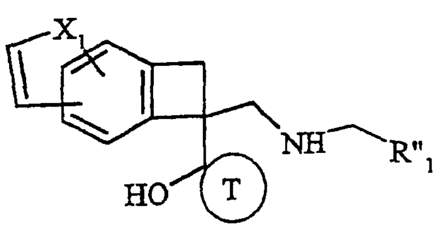

**(I/b$_3$)**

in der $X_1$, $R''_1$ und T die oben angegebenen Bedeutungen besitzen,

wobei die Gesamtheit der Verbindungen der Formeln (I/b$_1$) und (I/b$_3$) die Verbindungen der Formel (I/b) bildet, welche Verbindungen der Formel (I/b) unter Anwendung der oben beschriebenen gleichen Bedingungen mit einer Verbindung der Formel **(VIIIc)** behandelt werden:

$R'_2 - Z_1$ **(VIIIc)**

in der $Z_1$ die oben angegebenen Bedeutungen besitzt und $R'_2$ die gleichen Definitionen aufweist wie sie für $R_2$ bezüglich der Formel (I) angegeben sind mit Ausnahme der Bedeutung des Wasserstoffatoms,
zur Bildung der Verbindungen der Formel **(I/c),** einem Sonderfall der Verbindungen der Formel (I):

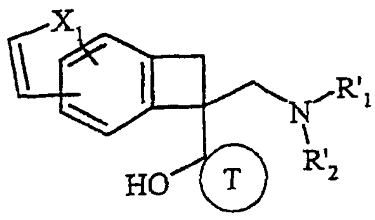

(I/c)

in der $X_1$, R, $R'_1$ und $R'_2$ die oben angegebenen Bedeutungen besitzen,
wobei die Gesamtheit der Verbindungen der Formeln (I/a), (I/b) und (I/c) in dem Fall, da $X_1$ ein Schwefelatom bedeutet, die Verbindungen der Formel **(I/e)** bildet:

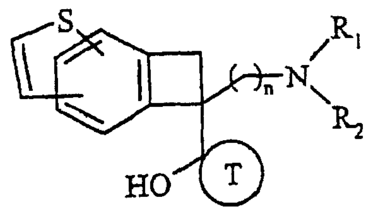

(I/e)

in der n, $R_1$, $R_2$ und T die bezüglich der Formel (I) angegebenen Bedeutungen besitzen,
welche Verbindungen der Formel (I/e) man der Einwirkung eines Oxidationsmittels unter Anwendung klassischer Bedingungen der organischen Synthese unterwirft zur Bildung der Verbindungen der Formel **(I/f),** einem Sonderfall der Verbindungen der Formel (I):

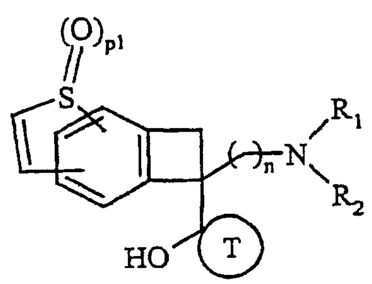

(I/f)

in der $p_1$ eine ganze Zahl ausgewählt aus 1 und 2 darstellt und n, $R_1$, $R_2$ und T die oben angegebenen Bedeutungen besitzen,
wobei die Gesamtheit der Verbindungen der Formeln (I/a), (I/b), (I/c) (bei denen $X_1$ ein Sauerstoff- oder Schwefelatom darstellt) und (I/f) die Verbindungen der Formel **(I/g)** bildet:

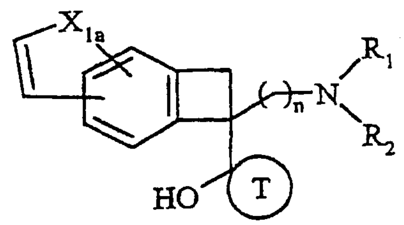

**(I/g)**

in der $X_{1a}$ ein Sauerstoffatom oder eine Gruppe der Formel $S(O)_p$ bedeutet, worin p die bezüglich der Formel (I) angegebenen Bedeutungen besitzt und n, $R_1$, $R_2$ und T die bezüglich der Formel (I) angegebenen Bedeutungen besitzen, welche Verbindungen der Formel (I/g) der Einwirkung eines Reduktionsmittels unter Anwendung klassischer Bedingungen der organischen Synthese unterworfen werden zur Bildung der Verbindungen der Formel **(I/h),** einem Sonderfall der Verbindungen der Formel (I):

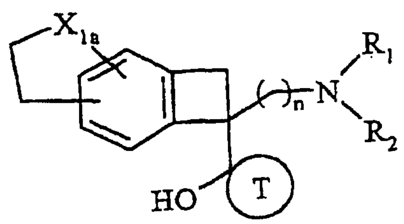

**(I/h)**

in der $X_{1a}$, n, $R_1$, $R_2$ und T die oben angegebenen Bedeutungen besitzen,

- oder dann, wenn $X_a$ die Bedeutung $X'_2$ annimmt, welche eine Gruppe NH darstellt, der Einwirkung eines Sulfonsäurechlorids der Formel **(XVI)** unterworfen werden:

$$E - SO_2Cl \qquad \textbf{(XVI)}$$

in der E eine geradkettige oder verzweigte ($C_1$-$C_4$)-Alkylgruppe, Phenylgruppe oder p-Toluylgruppe bedeutet, zur Bildung der Verbindungen der Formel **(XVII):**

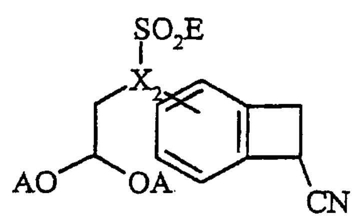

**(XVII)**

in der $X_2$ ein Stickstoffatom bedeutet und E und A die oben angegebenen Bedeutungen besitzen, welche Verbindungen der Formel (XVII) durch Einwirkung einer Säure cyclisiert werden zur Bildung der Verbindungen der Formel **(V/b):**

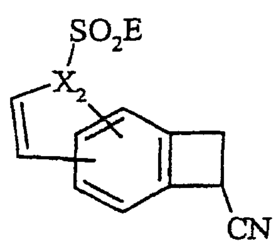

**(V/b)**

in der $X_2$ und E die oben angegebenen Bedeutungen besitzen,
von welchen Verbindungen der Formel (V/b) man die Schutzgruppe des cyclischen Amins durch Einwirkung eines basischen Mittels abspaltet und dann der Einwirkung eines Reduktionsmittels unter Anwendung klassischer Bedingungen der organischen Synthese unterwirft zur Bildung der Verbindungen der Formel **(V/c):**

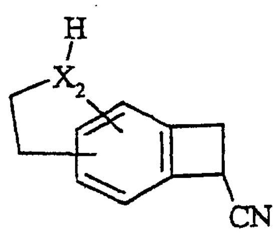

(V/c)

in der $X_2$ die oben angegebenen Bedeutungen besitzt,
welche Verbindungen der Formel (V/c) man der Einwirkung einer Verbindung der Formel (VI) unterwirft, wie sie oben beschrieben worden ist, zur Bildung der Verbindungen der Formel **(VII/b):**

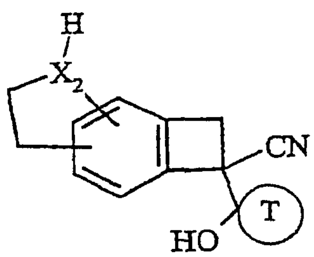

(VII/b)

in der $X_2$ die oben angegebenen. Bedeutungen besitzt und T die bezüglich der Formel (I) angegebenen Bedeutungen aufweist,
welche Verbindungen der Formel (VII/b) man:

- ***entweder*** man unter den gleichen Bedingungen behandelt, wie sie für die Verbindungen der Formel (VII/a) beschrieben worden sind, zur Bildung der Verbindungen der Formel **(I/i),** einem Sonderfall der Verbindungen der Formel (I):

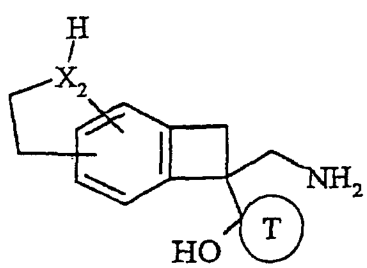

(I/i)

in der $X_2$ und T die oben angegebenen Bedeutungen besitzen,
welche Verbindungen der Formel (I/i) nacheinander mit einer Verbindung der Formel (VIIIa) oder (VIIb) und dann (VIIIc), wie sie oben definiert worden sind, behandelt werden können zur Bildung der Verbindungen der Formel **(I/j)** bzw. **(I/k),** Sonderfällen der Verbindungen der Formel (I):

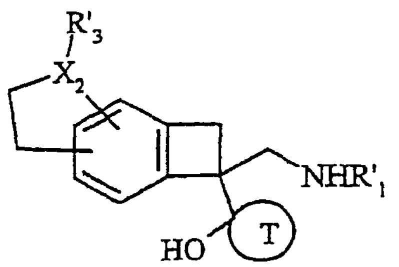

,

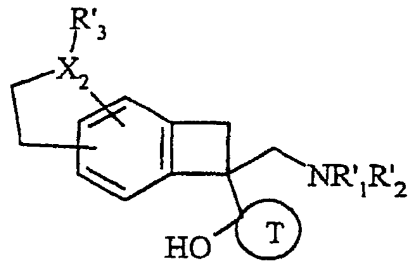

(I/j) (I/k)

in denen $X_2$, T, $R'_1$ und $R'_2$ die oben angegebenen Bedeutungen besitzen und $R'_3$ die gleichen Bedeutungen und Werte aufweist wie $R'_1$,
wobei die Gesamtheit der Verbindungen der Formeln (I/i), (I/j) und (I/k) die Verbindungen der Formel **(I/1)** bildet:

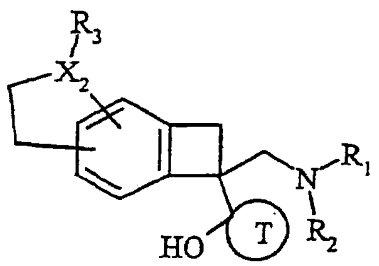

(I/l)

in der $X_2$, $R_1$, $R_2$, $R_3$ und T die bezüglich der Formel (I) angegebenen Bedeutungen besitzen,
welche Verbindungen der Formel (I/l) man der Einwirkung eines Oxidationsmittels, wie Mangandioxid, unterwirft zur Bildung der Verbindungen der Formel **(I/m),** einem Sonderfall der Verbindungen der Formel (I):

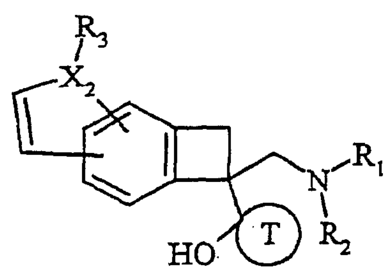

(I/m)

in der $X_2$, $R_1$, $R_2$, $R_3$ und T die oben angegebenen Bedeutungen besitzen,

- ***oder*** mit einer Verbindung der Formel (XVI), wie sie oben definiert worden ist, behandelt zur Bildung der Verbindungen der Formel **(XVIII):**

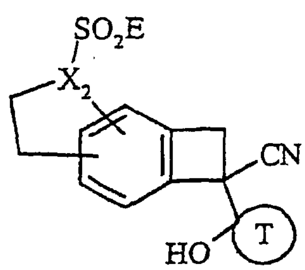

(XVIII)

in der T, $X_2$ und E die oben angegebenen Bedeutungen besitzen,
welche Verbindungen der Formel (XVIII) unter Anwendung klassischer Bedingungen der organischen Synthese reduziert werden zur Bildung der Verbindungen der Formel **(XIX):**

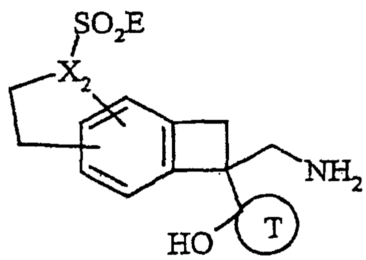

(XIX)

in der T, $X_2$ und E die oben angegebenen Bedeutungen besitzen,
bei welchen Verbindungen der Formel (XIX) die primäre Aminfunktion durch Einwirkung einer Verbindung der Formel ($VIII_a$), wie sie oben definiert worden ist, substituiert werden kann zur Bildung der Verbindungen der Formel **(XX):**

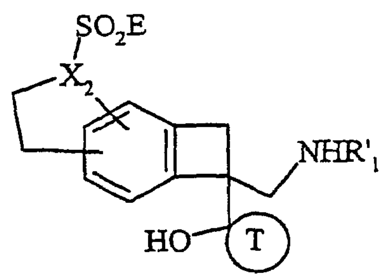

(XX)

in der T, $X_2$, E und $R'_1$ die oben angegebenen Bedeutungen besitzen,
welche Verbindungen der Formel (XX) durch Einwirkung einer Verbindung der Formel ($VIII_b$), wie sie oben definiert worden ist, in das tertiäre Amin umgewandelt werden können zur Bildung der Verbindungen der Formel **(XXI):**

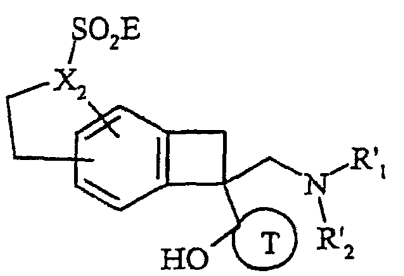

(XXI)

in der T, $X_2$, E, $R'_1$ und $R'_2$ die oben angegebenen Bedeutungen besitzen,
von welchen Verbindungen der Formeln (XX) und (XXI) anschließend die Schutzgruppen durch Behandeln mit Natrium in flüssigem Ammoniak abgespalten werden können, so daß man die Verbindungen der Formeln **(I/n)** bzw. **(I/o)** erhält, Sonderfällen der Verbindungen der Formel (I):

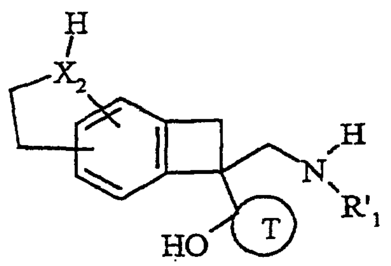

,

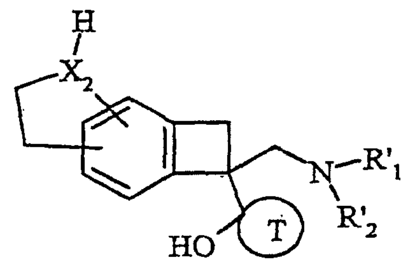

**(I/n)** **(I/o)**

in denen T, $X_2$, $R'_1$ und $R'_2$ die oben angegebenen Bedeutungen besitzen,

**b)** ***oder*** eine Verbindung der Formel **(II/1)** verwendet:

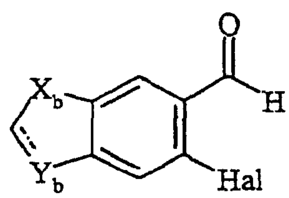

**(II/1)**

in der Hal ein Halogenatom und $X_b$ ein Sauerstoffatom bedeutet, wenn $Y_b$ ein Sauerstoffatom darstellt und ----- eine Einfachbindung bedeutet,
oder $X_b$ eine Gruppe -CH=CH- darstellt, wenn $Y_b$ eine Gruppe CH bedeutet und das Symbol ----- eine Doppelbindung darstellt,
welche Verbindungen der Formel (II/1) mit $(EtO)_2POCH_2CN$ umgesetzt wird zur Bildung der Verbindungen der Formel (II/2):

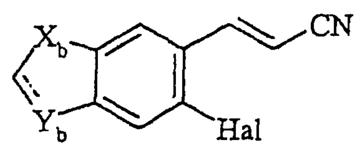

**(II/2)**

in der Hal, $X_b$ und $Y_b$ die oben angegebenen Bedeutungen besitzen,
welche Verbindungen der Formel (II/2) zunächst der Einwirkung eines klassischen Reduktionsmittels der organischen Chemie unterworfen werden und dann mit $NaNH_2$ umgesetzt werden zur Bildung der Verbindungen der Formel (II/3):

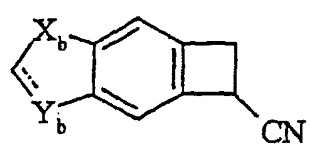

**(II/3)**

in der $X_b$ und $Y_b$ die oben angegebenen Bedeutungen besitzen,
welche Verbindungen der Formel (II/3) unter den gleichen Bedingungen wie die Verbindungen der Formel (V/a) mit einer Verbindung der Formel (VI) und dann (VIIIa) oder (VIIIb) und dann (VIIIc) behandelt werden können zur aufeinanderfolgenden Bildung der Verbindungen der Formel ($I/3_a$), ($I/3_b$) und ($I/3_c$):

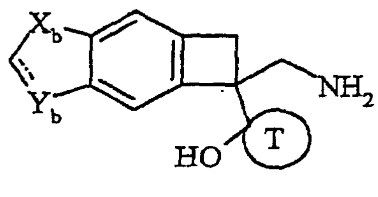 , 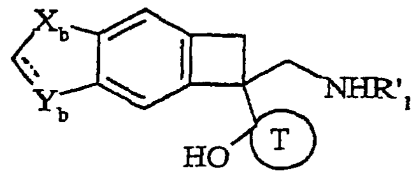

(I/3a) (I/3b)

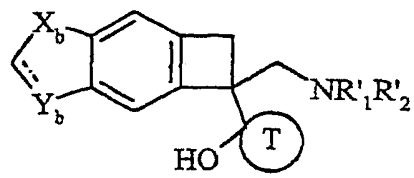

(I/3c)

worin $X_b$, $Y_b$, T, $R'_1$ und $R'_2$ die oben angegebenen Bedeutungen besitzen,
wobei die Verbindungen (I/a) bis (I/o) und (I/$3_a$) bis (I/$3_c$) die Gesamtheit der Verbindungen der Erfindung bilden, welche man gegebenenfalls mit Hilfe einer klassischen Reinigungsmethode reinigt, welche gewünschtenfalls mit Hilfe einer klassischen Trennmethode in ihre verschiedenen Isomeren aufgetrennt werden können und welche man gegebenenfalls in ihre Additionssalze mit einer pharmazeutisch annehmbaren Säure oder Base umwandelt.

**11.** Pharmazeutische Zubereitungen enthaltend als Wirkstoff mindestens eine Verbindung der Formel (I) nach einem der Ansprüche 1 bis 9 allein oder in Kombination mit einem oder mehreren inerten, nichttoxischen, pharmazeutisch annehmbaren Trägermaterialien oder Bindemitteln.

**12.** Pharmazeutische Zubereitungen nach Anspruch 11 enthaltend mindestens einen Wirkstoff nach einem der Ansprüche 1 bis 9 nützlich als Arzneimittel für die Behandlung von Depressionen, Panikanfällen, krampfartigen Besessenheitsstörungen, Phobien, impulsiven Störungen, Drogenmißbrauch, Angst, Fettsucht und Bulimie.

## Claims

**1.** Compounds of formula (I) :

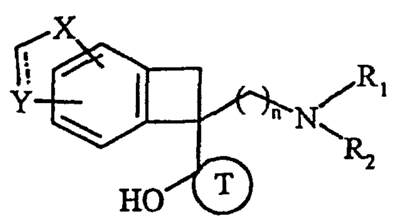

(I)

wherein :

----- denotes a single bond or a double bond,

n is 1,

$R_1$ and $R_2$, which may be identical or different, each independently of the other represent a group selected from a hydrogen atom, a linear or branched ($C_1$-$C_6$)alkyl group and 2,3-dihydro-1,4-benzodioxin-2-ylmethyl,

X represents a group selected from -CH=CH-, an oxygen atom, a group $S(O)_m$ wherein m is an integer from 0 to 2 inclusive, and $NR_3$ wherein $R_3$ represents a group selected from a hydrogen atom, a linear or branched $(C_1-C_6)$alkyl group, an aryl group, an aryl-$(C_1-C_6)$-alkyl group in which the alkyl moiety is linear or branched, a cycloalkyl group, a cycloalkyl-$(C_1-C_6)$alkyl group in which the alkyl moiety is linear or branched, a linear or branched $(C_2-C_6)$alkenyl group, and a linear or branched $(C_2-C_6)$alkynyl group,

Y represents a CH or $CH_2$ group depending on whether -------- denotes a single bond or a double bond, or may have the additional meaning of an oxygen atom when X represents an oxygen atom,

T represents a cyclopentyl or cyclohexyl group,

their optical isomers and addition salts thereof with a pharmaceutically acceptable acid or base,

it being understood that :

- "aryl group" is understood to mean a group selected from phenyl, biphenyl, naphthyl, dihydronaphthyl, tetrahydronaphthyl, indanyl, indenyl and benzocyclobutyl, it being possible for each of those groups to be optionally substituted by one or more identical or different groups selected from halogen atoms, linear or branched $(C_1-C_6)$alkyl groups, hydroxy groups, linear or branched $(C_1-C_6)$alkoxy groups, nitro groups, cyano groups, linear or branched trihalo-$(C_1-C_6)$alkyl groups, amino groups, monoalkylamino groups in which the alkyl moiety has from 1 to 6 carbon atoms and is linear or branched and dialkylamino groups in which each alkyl moiety has from 1 to 6 carbon atoms and is linear or branched,
- "cycloalkyl group" is understood to mean a mono- or poly-cyclic system, having from 3 to 12 ring members, optionally containing one or more unsaturated bonds, which do not confer an aromatic character upon the said ring system.

**2.** Compounds of formula (I) according to claim 1, **characterised in that** they are compounds of formula **(I/A) :**

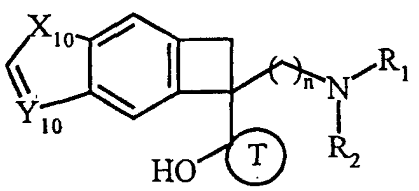

**(I/A)**

wherein n, $R_1$, $R_2$ and T are as defined for formula (I), $X_{10}$ represents an oxygen atom or a sulphur atom, and $Y_{10}$ represents a CH group, their isomers and addition salts thereof with a pharmaceutically acceptable acid or base.

**3.** Compounds of formula (I) according to claim 1, **characterised in that** they are compounds of formula **(I/B) :**

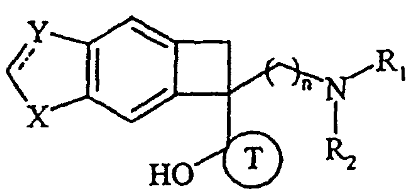

**(I/B)**

wherein X, Y, n, $R_1$, $R_2$, $R_3$ and T are as defined for formula (I), their isomers and addition salts thereof with a pharmaceutically acceptable acid or base.

**4.** Compounds of formula (I/B) according to claim 3, **characterised in that** X represents a group $NR_3$ wherein $R_3$ represents a hydrogen atom or a linear or branched $(C_1-C_6)$alkyl group, their isomers and addition salts thereof with a pharmaceutically acceptable acid or base.

**5.** Compounds of formula (I) according to claim 1, **characterised in that** they are compounds of formula **(I/C) :**

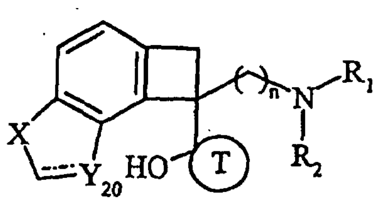

(I/C)

wherein n, $R_1$, $R_2$, X and T are as defined for formula (I) and $Y_{20}$ represents a CH or $CH_2$ group, their isomers and addition salts thereof with a pharmaceutically acceptable acid or base.

**6.** Compounds of formula (I) according to claim 1, **characterised in that** they are compounds of formula **(I/D) :**

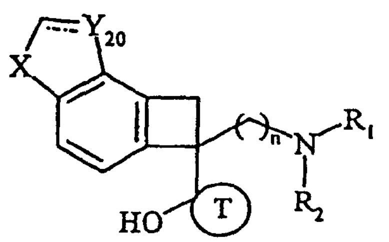

(I/D)

wherein n, $R_1$, $R_2$, X and T are as defined for formula (I) and $Y_{20}$ represents a CH or $CH_2$ group, their isomers and addition salts thereof with a pharmaceutically acceptable acid or base.

**7.** Compounds of formula (I) according to claim 1, **characterised in that** they are compounds of formula **(I/E) :**

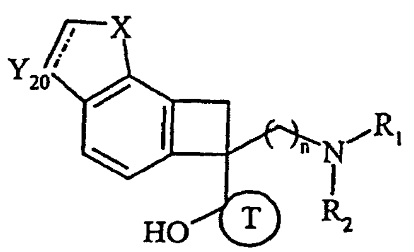

(I/E)

wherein n, $R_1$, $R_2$, X and T are as defined for formula (I) and $Y_{20}$ represents a CH or $CH_2$ group, their isomers and addition salts thereof with a pharmaceutically acceptable acid or base.

**8.** Compounds of formula (I) according to claim 1, **characterised in that** they are compounds of formula **(I/F) :**

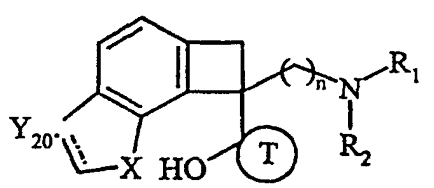

(I/F)

wherein n, $R_1$, $R_2$, X and Y are as defined for formula (I) and $Y_{20}$ represents a CH or $CH_2$ group, their isomers and addition salts thereof with a pharmaceutically acceptable acid or base.

**9.** Compounds of formula (I) according to claim 1 which are :

- 1-{6-[(dimethylamino)methyl]-1-methyl-2,3,5,6-tetrahydro-1*H*-cyclobuta[*f*]indol-6-yl} cyclohexanol,

- 1-{6-[(dimethylamino)methyl]-2,3,5,6-tetrahydro-1*H*-cyclobuta[*f*]indol-6-yl}-cyclohexanol,
- 1-{5-[(dimethylamino)methyl]-5,6-dihydrocyclobuta[*f*][1]benzofuran-5-yl}cyclohexanol,
- 1-{5-[(dimethylamino)methyl]-5,6-dihydrocyclobuta[*f*][*1*]benzothien-5-yl}-cyclohexanol,
- 1-{6-[(dimethylamino)methyl]-1-methyl-5,6-dihydro-1*H*-cyclobuta[*f*]indol-6-yl}-cyclohexanol,
- 1-{7-[(dimethylamino)methyl]-2,3,6,7-tetrahydro-1*H*-cyclobuta[*e*]indol-7-yl}-cyclohexanol,
- 1-{5-[(methylamino)methyl]-5,6-dihydrocyclobuta[*f*][1]benzothien-5-yl}-cyclopentanol,
- 1-{5-[(dimethylamino)methyl]-5,6-dihydrocyclobuta[*f*][1]benzothien-5-yl}-cyclopentanol,
- (+)-1-{5-[(dimethylamino)methyl]-5,6-dihydrocyclobuta[*f*][1]benzothien-5-yl}-cyclopentanol,
- (-)-1-{5-[(dimethylamino)methyl]-5,6-dihydrocyclobuta[*f*][1]benzothien-5-yl}-cyclopentanol,
- 1-{6-[(dimethylamino)methyl]-5,6-dihydrocyclobuta[*f*][1]benzothien-6-yl}-cyclopentanol,
- 1-{5-[(dimethylamino)methyl]-1-methyl-5,6-dihydro-1*H*-cyclobuta[*f*]indol-5-yl}-cyclopentanol,
- 1-{7-[(dimethylamino)methyl]-6,7-dihydrocyclobuta[*g*][1]benzofuran-7-yl}-cyclopentanol,
- 1-{1-[(dimethylamino)methyl]-1,2-dihydrocyclobuta[b]naphthalen-1-yl}cyclopentanol,
- 1-{7-[(dimethylamino)methyl]-6,7-dihydro-3*H*-cyclobuta[*e*]indol-7-yl}cyclopentanol,
- 1-{1-[(dimethylamino)methyl]-1,2-dihydrocyclobuta[*a*]naphthalen-1-yl}cyclopentanol, their isomers and addition salts thereof with a pharmaceutically acceptable acid or base.

**10.** Process for the preparation of compounds of formula (I), **characterised in that** there is used as starting material :

***a) <u>either</u>*** a compound of formula **(II) :**

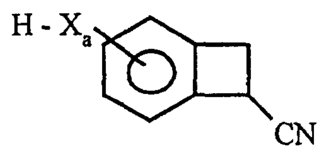

**(II)**

wherein $X_a$ represents a sulphur atom, an oxygen atom or an NH group,
which compound of formula (II) is reacted with a compound of formula **(III) :**

$$Z - CH(OA)_2 \qquad \textbf{(III)}$$

wherein A represents a linear or branched ($C_1$-$C_4$)alkyl group and Z represents a formyl group (when $X_a$ represents an NH group), or a group $-CH_2Hal$ wherein Hal represents a chlorine, bromine or iodine atom, to yield compounds of formula **(IV) :**

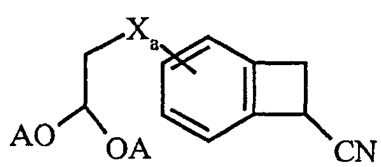

**(IV)**

wherein $X_a$ and A are as defined hereinbefore,
which compounds of formula (IV) are subjected :

- <u>either, when $X_a$ has the meaning $X_1$</u> representing an oxygen atom or a sulphur atom, to conditions of cyclisation by the action of an acid, such as polyphosphoric acid or a Lewis acid, to yield compounds of formula **(V/a) :**

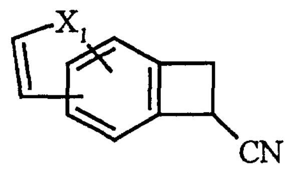

**(V/a)**

wherein $X_1$ represents an oxygen atom or a sulphur atom, which compounds of formula (V/a) are treated in the presence of a strong base, with a cyclic ketone of formula **(VI)** :

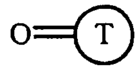

**(VI)**

wherein T has the same meanings as for formula (I), to yield compounds of formula **(VII/a),**

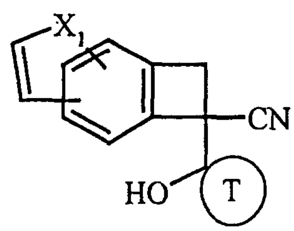

**(VII/a)**

wherein $X_1$ and T are as defined hereinbefore, which compounds of formula (VII/a) are subjected to the action of a reducing agent according to conventional conditions of organic synthesis, to yield compounds of formula **(I/a),** a particular case of the compounds of formula (I) :

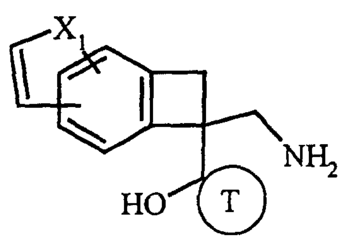

**(I/a)**

wherein $X_1$ and T are as defined hereinbefore, for which compounds of formula (I/a) :

⇨ ***either*** the primary amine function is substituted according to conventional methods of organic synthesis, such as reductive amination or nucleophilic substitution with a compound of formula **(VIIIa) :**

$$R'_1 - Z_1 \qquad \textbf{(VIIIa)}$$

wherein $R'_1$ has the same meanings as $R_1$ for formula (I) with the exception of the meaning of a hydrogen atom, and $Z_1$ represents a leaving group customary in organic chemistry, such as a halogen atom or a mesylate or tosylate group, to yield compounds of formula **($I/b_1$),** a particular case of the compounds of formula (I) :

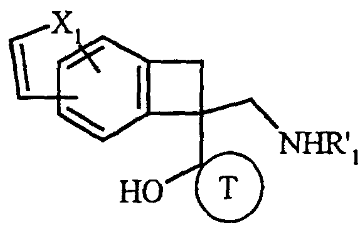

**(I/b$_1$)**

wherein $X_1$, T and $R'_1$ are as defined hereinbefore,

⇨ ***or*** according to an advantageous embodiment of the process, treatment is carried out with a compound of formula **(VIIIb) :**

$$R''_1 - COZ_2 \quad \textbf{(VIIIb)}$$

wherein $R''_1$ represents a heterocycloalkyl-($C_1$-$C_5$)alkyl group in which the alkyl moiety is linear or branched, and $Z_2$ represents a chlorine atom or an imidazolyl group, to yield compounds of formula **(I/b$_2$) :**

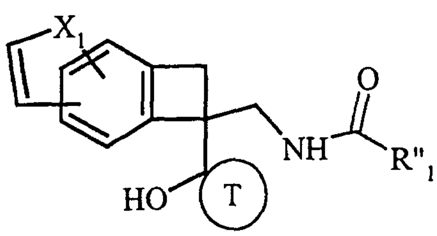

**(I/b$_2$)**

wherein $X_1$, T and $R''_1$ are as defined hereinbefore, which compounds of formula (I/b$_2$) are reduced with a reducing agent conventionally used in organic chemistry, to yield compounds of formula **(I/b$_3$) :**

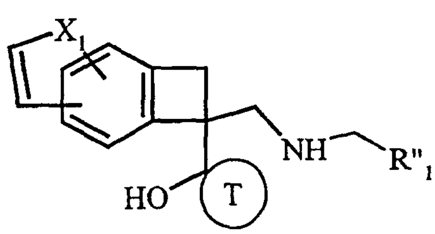

**(I/b$_3$)**

wherein $X_1$, $R''_1$ and T are as defined hereinbefore, the totality of the compounds of formulae (I/b$_1$) and (I/b$_3$) constituting the compounds of formula (I/b), which compounds of formula (I/b) are treated according to the same conditions as those described hereinbefore with a compound of formula **(VIIIc) :**

$$R'_2 - Z_1 \quad \textbf{(VIIIc)}$$

wherein $Z_1$ is as defined hereinbefore and $R'_2$ has the same meanings as $R_2$ for formula (I) with the exception of the meaning of a hydrogen atom, to yield compounds of formula **(I/c),** a particular case of the compounds of formula (I) :

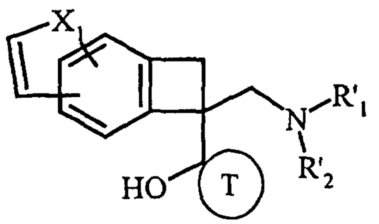

(I/c)

wherein $X_1$, T, $R'_1$ and $R'_2$ are as defined hereinbefore,
the totality of the compounds of formulae (I/a), (I/b) and (I/c), in the particular case when $X_1$ represents a sulphur atom, constituting the compounds of formula **(I/e) :**

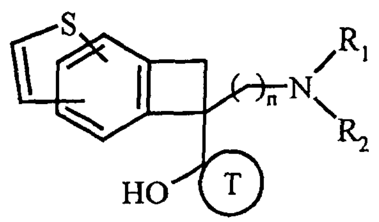

(I/e)

wherein n, $R_1$, $R_2$ and T are as defined for formula (I),
which compounds of formula (I/e) are subjected to the action of an oxidising agent according to conventional conditions of organic synthesis, to yield compounds of formula **(I/f),** a particular case of the compounds of formula (I) :

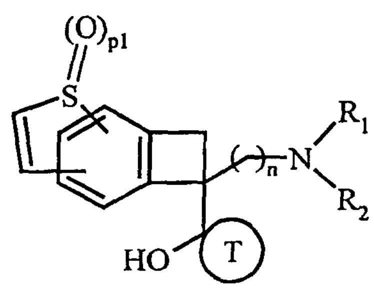

(I/f)

wherein $p_1$ is an integer selected from 1 and 2, and n, $R_1$, $R_2$ and T are as defined hereinbefore,
the totality of the compounds of formulae (I/a), (I/b), (I/c) (wherein $X_1$ represents an oxygen or sulphur atom) and (I/f) constituting the compounds of formula (I/g) :

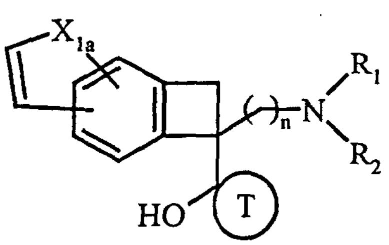

(I/g)

wherein $X_{1a}$ represents an oxygen atom or a group of formula $S(O)_p$ wherein p is as defined for formula (I), and n, $R_1$, $R_2$ and T are as defined for formula (I),
which compounds of formula (I/g) are subjected to the action of a reducing agent according to conventional conditions of organic synthesis, to yield compounds of formula **(I/h),** a particular case of the compounds of formula (I) :

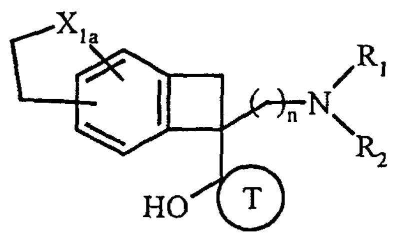

(I/h)

wherein $X_{1a}$, n, $R_1$, $R_2$ and T are as defined hereinbefore,

- or, when $X_a$ has the meaning $X'_2$ representing an NH group, to the action of a sulphonic acid chloride of formula **(XVI)** :

E - $SO_2Cl$ **(XVI)**

wherein E represents a linear or branched ($C_1$-$C_4$)alkyl group, a phenyl group or a p-toluyl group, to yield compounds of formula **(XVII)** :

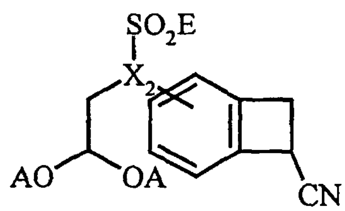

(XVII)

wherein $X_2$ represents a nitrogen atom, and E and A are as defined hereinbefore, which compounds of formula (XVII) are cyclised by the action of an acid to yield compounds of formula **(V/b)** :

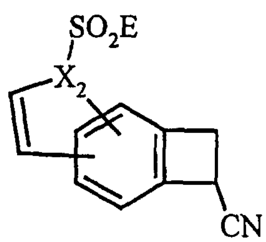

(V/b)

wherein $X_2$ and E are as defined hereinbefore, the cyclic amine of which compounds of formula (V/b) is deprotected by the action of a basic agent, which are then subjected to the action of a reducing agent according to conventional conditions of organic synthesis, to yield compounds of formula **(V/c)** :

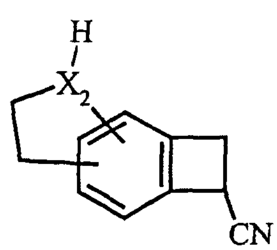

(V/c)

wherein $X_2$ is as defined hereinbefore,
which compounds of formula (V/c) are subjected to the action of a compound of formula (VI) as described hereinbefore to yield compounds of formula **(VII/b) :**

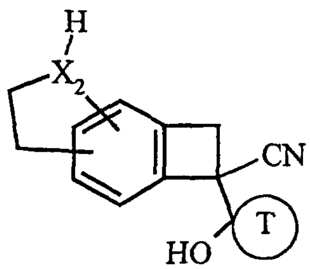

**(VII/b)**

wherein $X_2$ is as defined hereinbefore and T is as defined for formula (I),
which compounds of formula (VII/b) are :

- ***either*** treated according to the same conditions as those described for the compounds of formula (VII/a), to yield compounds of formula **(I/i),** a particular case of the compounds of formula (I):

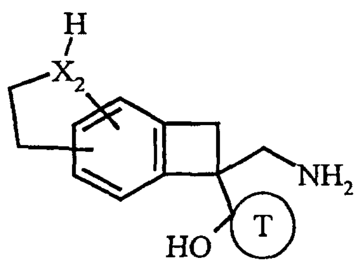

**(I/i)**

wherein $X_2$ and T are as defined hereinbefore,
which compounds of formula (I/i) may be treated in succession with a compound of formula (VIIIa) or (VIIIb), and then (VIIIc), as defined hereinbefore, to yield compounds of formulae **(I/j)** and **(I/k),** respectively, particular cases of the compounds of formula **(I)** :

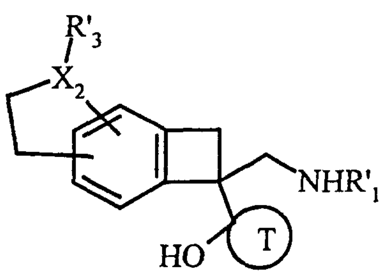

,

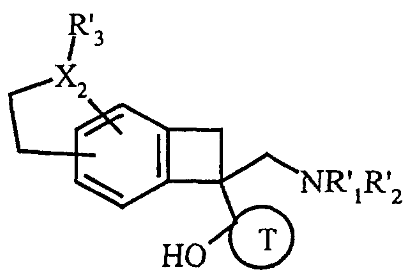

**(I/j)** **(I/k)**

wherein $X_2$, T, $R'_1$ and $R'_2$ are as defined hereinbefore, and $R'_3$ has the same meanings and values as $R'_1$,
the totality of the compounds of formulae (I/i), (I/j) and (I/k) constituting the compounds of formula **(I/l) :**

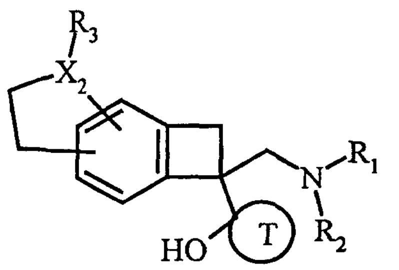

(I/l)

wherein $X_2$, $R_1$, $R_2$, $R_3$ and T are as defined for formula (I),
which compounds of formula (I/l) are subjected to the action of an oxidising agent, such as manganese dioxide, to yield compounds of formula **(I/m),** a particular case of the compounds of formula (I) :

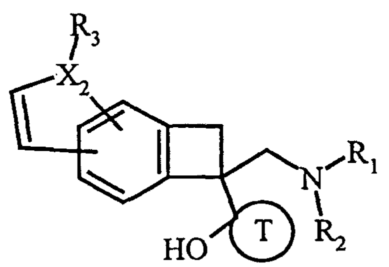

(I/m)

wherein $X_2$, $R_1$, $R_2$, $R_3$ and T are as defined hereinbefore,

- ***or*** treated with a compound of formula (XVI) as defined hereinbefore, to yield compounds of formula **(XVIII) :**

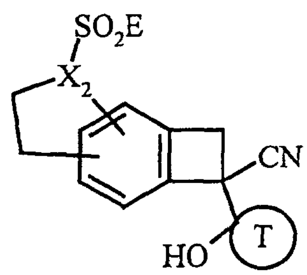

(XVIII)

wherein T, $X_2$ and E are as defined hereinbefore,
which compounds of formula (XVIII) are reduced according to conventional conditions of organic synthesis, to yield compounds of formula **(XIX) :**

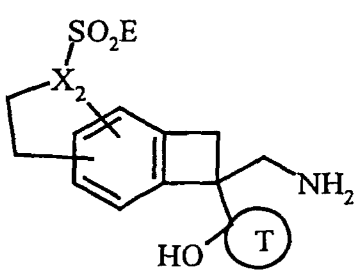

(XIX)

wherein T, $X_2$ and E are as defined hereinbefore,
the primary amine function of which compounds of formula (XIX) may be substituted by the action of a compound of formula ($VIII_a$) as defined hereinbefore, to yield compounds of formula **(XX) :**

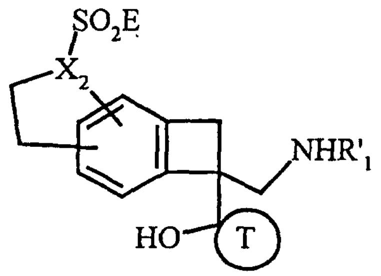

**(XX)**

wherein T, $X_2$, E and $R'_1$ have the same meanings as those described hereinbefore, which compounds of formula (XX) may be converted into tertiary amines by the action of a compound of formula ($VIII_b$) as defined hereinbefore, to yield compounds of formula **(XXI) :**

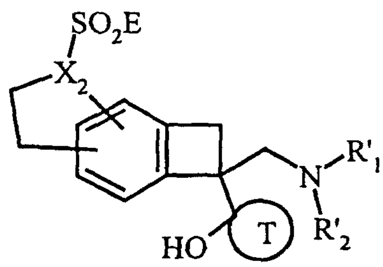

**(XXI)**

wherein T, $X_2$, E, $R'_1$ and $R'_2$ are as defined hereinbefore, which compounds of formulae (XX) and (XXI) are then deprotected by treatment with sodium in liquid ammonia, to yield compounds of formulae **(I/n)** and **(I/o),** respectively, particular cases of the compounds of formula (I) :

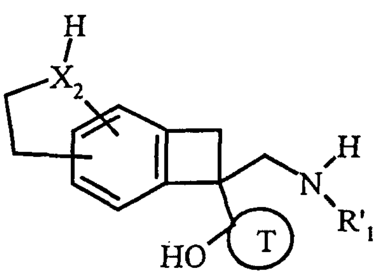

,

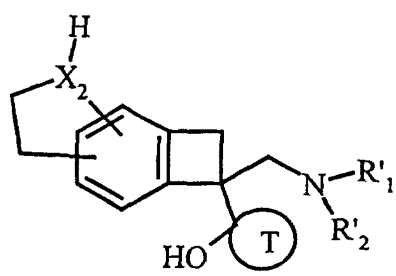

**(I/n)** **(I/o)**

wherein T, $X_2$, $R'_1$ and $R'_2$ are as defined hereinbefore,

**b) *<u>or</u>*** a compound of formula **(II/1) :**

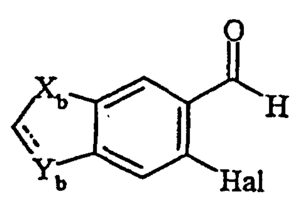

**(II/1)**

wherein Hal represents a halogen atom, and $X_b$ represents an oxygen atom when $Y_b$ represents an oxygen atom and <u>-----</u> denotes a single bond,

or $X_b$ represents a group -CH=CH- when $Y_b$ represents a CH group and ----- denotes a double bond, which compounds of formula (II/1) are reacted with $(EtO)_2POCH_2CN$ to yield compounds of formula (II/2) :

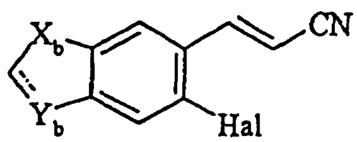

(II/2)

wherein Hal, $X_b$ and $Y_b$ are as defined hereinbefore,
which compounds of formula (II/2) are first subjected to the action of a reducing agent conventional in organic chemistry and then reacted with $NaNH_2$, to yield compounds of formula (II/3) :

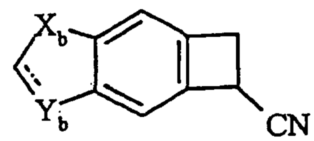

(II/3)

wherein Xb and Yb are as defined hereinbefore,
which compounds of formula (II/3) may be treated under the same conditions as the compounds of formula (V/a), with a compound of formula (VI), then (VIIIa) or (VIIIb), and then (VIIIc), to yield, in succession, compounds of formulae ($I/3_a$), ($I/3_b$) and ($I/3_c$)

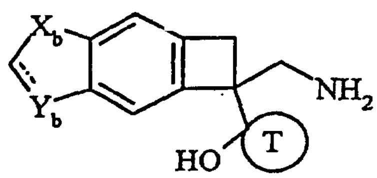

,

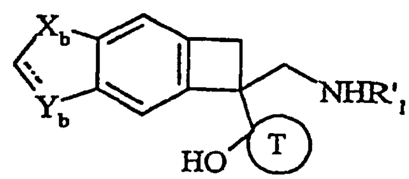

($I/3_a$) ($I/3_b$)

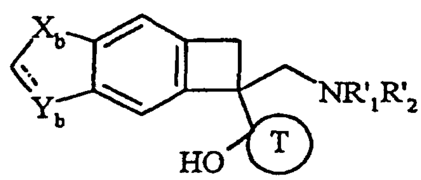

($I/3_c$)

wherein $X_b$, $Y_b$, T, $R'_1$ and $R'_2$ are as defined hereinbefore,
which compounds (I/a) to (I/o) and ($I/3_a$) to ($I/3_c$) constitute the totality of the compounds of the invention, which are purified, if necessary, according to a conventional purification technique, which may be separated, if desired, into their different isomers according to a conventional separation technique, and which are converted, where appropriate, into their addition salts with a pharmaceutically acceptable acid or base.

**11.** Pharmaceutical compositions comprising as active ingredient at least one compound of formula (I) according to any one of claims 1 to 9, alone or in combination with one or more inert, non-toxic pharmaceutically acceptable excipients or carriers.

**12.** Pharmaceutical compositions according to claim 11 comprising at least one active ingredient according to any one of claims 1 to 9, for use as medicaments in the treatment of depression, panic attacks, obsessive-compulsive

disorders, phobias, impulsive disorders, drug abuse, anxiety, obesity and bulimia.